# EUROPEAN PATENT APPLICATION

(11) **EP 2 392 332 A1**
(43) Date of publication of application: **07.12.2011**
(21) Application number: 10184480.1
(22) Date of filing: 07.10.2005
(51) Int. Cl.: A61K 31/56, A61K 31/565, A61K 31/567, A61P 15/18

(54) **Methods of hormonal treatment utilizing ascending-dose extended cycle regimens**

(30) Priority: 07.10.2004 US 616424 P; 26.05.2005 US 684568 P
(62) Divisional of application: 05810167.6
(71) Applicant: Teva Women's Health, Inc., Cincinnati, OH 45213-2520 (US)
(72) Inventor: Diliberti, Charles, E., Montclair, NJ 07043 (US); Reape, Kathleen, Z., Bryn Mawr, PA 19010 (US); Bronnenkant, Lance, J, Snyder, NY 14226 (US)
(74) Representative: Clarke, Lionel Paul

(57) **Abstract**

The present invention provides ascending-dose extended cycle regimens in which a female is administered an estrogen and a progestin for a period greater than 30 or 31 consecutive days, optionally followed by a hormone-free period or by a period of administration of estrogen. The disclosed regimens can be administered to a female to provide contraceptive and non-contraceptive benefits.

## Description

### Background of the Invention

### Field of the Invention

The present invention relates to methods of hormonal treatment by ascending-dose extended administration of an estrogen and a progestin.

### Related Art

The human menstrual cycle involves a repetitive sequence of hormonal changes that result in episodic uterine bleeding. Normally, each menstrual cycle has a mean interval of 21 to 35 days, conventionally beginning with the first day of menstrual flow and ending on the day before the next onset of bleeding. Duration of the menstrual flow is usually 2 to 6 days with loss of about 20 to about 60 ml of blood.

The menstrual cycle is divided into follicular and luteal phases, each corresponding to changes occurring in the ovary. These phases may also be described as proliferative or secretory, corresponding to changes observed in the uterine endometrium. Variations in the length of the cycle are usually due to alterations in the follicular phase, because the luteal phase length remains relatively constant at 12 to 16 days.

During the follicular phase, several primary follicles are recruited for further growth and development. Granulosa cells in primary follicles possess follicle stimulating hormone (FSH) and estradiol receptors. Upon FSH stimulation, granulosa cells produce aromatase. This enzyme converts the androgens androstenedione and testosterone, made in response to luteinizing hormone (LH) by thecal cells, to estrone and estradiol, respectively. Granulosa cells respond to estradiol by undergoing mitosis to increase the number of granulosa cells and estradiol production. By day 7 of the cycle, one enlarging primary follicle is selected by unknown processes to be the follicle that will release the oocyte at ovulation.

The midcycle rise in plasma estradiol stimulates the large midcycle LH surge. This midcycle LH surge triggers resumption of meiosis within the oocyte and luteinization of the granulosa cells within the preovulatory follicle. Immediately before ovulation, the outer follicular wall begins to dissolve and an oocyte is released approximately 24 to 36 hours from the onset of the LH surge.

After ovulation, granulosa cells and the surrounding theca cells enlarge, accumulate lipid, and become transformed into lutein cells. This begins the luteal phase of the menstrual cycle. These cells form a new vascularized structure called the corpus luteum, which secretes estradiol and progesterone. LH maintains the corpus luteum during the luteal phase and, acting via the adenyl cyclase system, stimulates progesterone production. If pregnancy does not occur, lutein cells degenerate, and diminished hormone secretion precedes menstruation. Menstruation is immediately followed by the onset of another menstrual cycle.

Because endometrial proliferation serves to prepare the uterus for an impending pregnancy, manipulation of hormones and of the uterine environment can provide contraception. For example, estrogens are known to decrease FSH secretion by feedback inhibition. Under certain circumstances, estrogens can also inhibit LH secretion, once again by negative feedback. Under normal circumstances, the spike of circulating estrogen found just prior to ovulation induces the surge of gonadotropic hormones that occurs just prior to and results in ovulation. High doses of estrogen immediately post-coitally also can prevent conception probably due to interference with implantation.

Progestins can also provide contraception. Endogenous progesterone after estrogen is responsible for the progestational changes of the endometrium and the cyclic changes of cells and tissue in the cervix and the vagina. Administration of progestin makes the cervical mucus thick, tenacious and cellular which is believed to impede spermatozoal transport. Administration of progestin also inhibits luteinizing hormone secretion and blocks ovulation in humans.

The most prevalent form of oral contraception is a pill that combines both an estrogen and a progestin, a so-called combined oral contraceptive preparation. Alternatively, there are contraceptive preparations that comprise progestin only. However, the progestin-only preparations have a more varied spectrum of side effects than do the combined preparations, especially more breakthrough bleeding. As a result, the combined preparations are the preferred oral contraceptives in use today (Sheth et al., Contraception 25:243 (1982)).

Whereas the conventional 21 day pill packs with a 7 day placebo interval worked well when oral contraceptives were of higher dosage, as the doses have come down, for both the estrogen and progestin components, bleeding problems have increased in frequency, especially in the early months of oral contraceptive use, but even persistently so in some patients.

There exists a need for contraceptives that reduce bleeding problems and/or have additional benefits for women.

### Brief Summary of the Invention

The present invention provides ascending-dose extended cycle regimens in which a female is administered an estrogen and a progestin for a period of greater than 30 or 31 consecutive days, optionally followed by a hormone-free period of 2 to 10 consecutive days or by administration of estrogen for a period of 2 to 10 consecutive days.

The present invention is directed to a method of contraception, the method comprising administering to a female in need thereof an estrogen and a progestin for a period of greater than 30 or 31 consecutive days, wherein the estrogen and progestin are administered in at least three phases, wherein a daily dosage of estrogen in a second phase is equal to or higher than a daily dosage of estrogen in a first phase, wherein a daily dosage of estrogen in a third phase is equal to or higher than the daily dosage of estrogen in the second phase, wherein a total daily dosage of estrogen and progestin in the second phase is higher than a total daily dosage of estrogen and progestin in the first phase, and wherein a total daily dosage of estrogen and progestin in the third phase is higher than the total daily dosage of estrogen and progestin in the second phase.

The present invention is also directed to a method of contraception, the method comprising administering to a female in need thereof an estrogen and a progestin for a period of greater than 30 or 31 consecutive days, wherein the estrogen and progestin are administered in at least two phases, wherein a total daily dosage of estrogen and progestin in a second phase is higher than a total daily dosage of estrogen and progestin in a first phase, and wherein a daily dosage of progestin in the second phase is less than twice a daily dosage of progestin in the first phase.

The ascending-dose extended cycle regimens provide a number of non-contraceptive benefits, as well as contraceptive benefits. For example, the present invention is also directed to a method of reducing breakthrough bleeding in a female in need thereof by administering to the female an ascending-dose extended cycle regimen disclosed herein.

### Detailed Description of the Invention

The present invention is directed to ascending-dose extended cycle regimens that are useful in the treatment of a variety of conditions and disorders occurring in females of child-bearing age, in peri-menopausal females, and/or in menopausal females. In accordance with the present invention, a female is administered an ascending-dose extended cycle regimen of an estrogen and a progestin (or progestogen) for a period of greater than 30 or 31 consecutive days.

An "ascending-dose extended cycle regimen" of the present invention refers to a regimen disclosed herein in which an estrogen and a progestin are administered for a period of greater than 30 or 31 consecutive days, wherein the estrogen and progestin are administered in at least three phases, wherein a daily dosage of estrogen in a second phase is equal to or higher than a daily dosage of estrogen in a first phase, wherein a daily dosage of estrogen in a third phase is equal to or higher than the daily dosage of estrogen in the second phase, wherein a total daily dosage of estrogen and progestin in the second phase is higher than a total daily dosage of estrogen and progestin in the first phase, and wherein a total daily dosage of estrogen and progestin in the third phase is higher than the total daily dosage of estrogen and progestin in the second phase.

An "ascending-dose extended cycle regimen" of the present invention also refers to a regimen disclosed herein in which an estrogen and a progestin are administered for a period of greater than 30 or 31 consecutive days, wherein the estrogen and progestin are administered in at least two phases, wherein a total daily dosage of estrogen and progestin in a second phase is higher than a total daily dosage of estrogen and progestin in a first phase, and wherein a daily dosage of progestin in the second phase is less than twice a daily dosage of progestin in the first phase.

As used herein, "extended cycle regimen" refers to a regimen in which a contraceptive composition is administered for a period of greater than 30 or 31 days.

As used herein, "female" refers to any animal classified as a mammal, including humans and non-humans, such as, but not limited to, domestic and farm animals, zoo animals, sports animals, and pets.

"Peri-menopausal female" refers to a woman who has not yet definitely arrived at menopause but who is experiencing symptoms associated with menopause. "Peri-menopause" means "about or around the time of menopause." It encompasses the years preceding the last menstrual period during which ovarian function declines and ultimately ceases and can include the presence of symptoms and irregular cycles. "Menopausal female" refers to a woman who has definitely arrived at menopause and may be experiencing symptoms associated with menopause. Menopause or post-menopause is the permanent cessation of menstruation after the loss of ovarian activity and is generally defined clinically as the absence of menstruation for about one year. Menopause may occur naturally in a woman or it may be artificially induced, e.g., through surgical or chemical means. For example, removal of the ovaries, which can occur, e.g., through hysterectomy, frequently leads to symptoms associated with menopause.

The terms "treat" and "treatment" refer to both therapeutic treatment and prophylactic or preventative measures, wherein the object is to prevent or slow down (lessen) an undesired physiological condition, disorder or disease, or obtain beneficial or desired clinical results. For purposes of this invention, beneficial or desired clinical results include, but are not limited to, alleviation of symptoms; diminishment of extent of condition, disorder or disease; stabilized (i.e., not worsening) state of condition, disorder or disease; delay in onset or slowing of condition, disorder or disease progression; amelioration of the condition, disorder or disease state, remission (whether partial or total), whether detectable or undetectable; or enhancement or improvement of condition, disorder or disease. Treatment includes eliciting a clinically significant response, without excessive levels of side effects. Treatment also includes prolonging survival as compared to expected survival if not receiving treatment.

The term "continuous" or "consecutive" in reference to "administration" means that the frequency of administration is at least once daily. Note, however, that the frequency of administration can be greater than once daily and still be "continuous," e.g., twice or even three times daily, as long as the dosage levels as specified herein are not exceeded.

The term "daily dosage," "daily dosage level," "daily dosage amount," or "daily dose" means the total amount of estrogen (and/or progestin) administered per day. Thus, for example, "continuous administration" of a progestin to a woman at a "daily dosage level" of 30 µg means that the woman receives a total of 30 µg of progestin on a daily basis, whether the progestin is administered as a single 30 µg dose or, e.g., three separate 10 µg doses. A conventional means of continuously administering an estrogen or progestin is as a single daily oral dose at the prescribed daily dosage level.

As used herein, "about" refers to plus or minus 10% of the indicated number. For example, "about 10 µg " indicates a range of 9 µg to 11 µg.

### Dosages and Regimens

The present invention is directed to a method of providing an ascending-dose extended cycle regimen, the method comprising administering to a female in need thereof an estrogen and a progestin for a period of greater than 30 or 31 consecutive days, wherein the estrogen and progestin are administered in at least three phases, wherein a daily dosage of estrogen in a second phase is equal to or higher than a daily dosage of estrogen in a first phase, wherein a daily dosage of estrogen in a third phase is equal to or higher than the daily dosage of estrogen in the second phase, wherein a total daily dosage of estrogen and progestin in the second phase is higher than a total daily dosage of estrogen and progestin in the first phase, and wherein a total daily dosage of estrogen and progestin in the third phase is higher than the total daily dosage of estrogen and progestin in the second phase.

The present invention is also directed to a method of providing an ascending-dose extended cycle regimen, the method comprising administering to a female in need thereof an estrogen and a progestin for a period of greater than 30 or 31 consecutive days, wherein the estrogen and progestin are administered in at least two phases, wherein a total daily dosage of estrogen and progestin in a second phase is higher than a total daily dosage of estrogen and progestin in a first phase, and wherein a daily dosage of progestin in the second phase is less than twice a daily dosage of progestin in the first phase.

In some aspects of the invention, the daily dosage of progestin in the first and second phases are equal to each other. In further aspects of the invention, the daily dosages of progestin in the first, second, and third phases are equal to each other. The daily dosage of progestin can be, but is not limited to, the equivalent of 150 µg of levonorgestrel for the first, second, and third phases.

In some aspects of the invention, the daily dosage of progestin in the second phase is higher than the daily dosage of progestin in the first phase. In further aspects of the invention, the daily dosage of progestin in the third phase is higher than the daily dosage of progestin in the second phase.

In some aspects of the invention, the daily dosage of progestin in the second phase is equal to the daily dosage of progestin in the first phase and the daily dosage of progestin in the third phase is higher than the daily dosage of progestin in the second phase. In other aspects of the invention, the daily dosage of progestin in the second phase is higher than the daily dosage of progestin in the first phase and the daily dosage of progestin in the third phase is equal to the daily dosage of progestin in the second phase.

In some aspects of the invention, the daily dosage of progestin in the second phase is less than twice the daily dosage of progestin in the first phase. In further aspects of the invention, the daily dosage of progestin in the third phase is less than twice the daily dosage of progestin in the second phase.

The daily dosage of progestin in the first phase can be, but is not limited to, the equivalent of 95 µg to 105 µg, 97 µg to 102 µg, or 99 µg to 101 µg of levonorgestrel. For example, the daily dosage of progestin in the first phase can be the equivalent of 100 µg levonorgestrel. The daily dosage of progestin in the second phase can be, but is not limited to, the equivalent of 120 µg to 130 µg, 122 µg to 128 µg, or 124 µg to 126 µg of levonorgestrel. For example, the daily dosage of progestin in the second phase can be the equivalent of 125 µg of levonorgestrel. The daily dosage of progestin in the third phase can be, but is not limited to, the equivalent of 145 µg to 155 µg, 147 µg to 153 µg, or 149 µg to 151 µg of levonorgestrel. For example, the daily dosage of progestin in the third phase can be the equivalent of 150 µg of levonorgestrel.

In some aspects of the invention, the progestin used is trimegestone. The total daily dosage of trimegestone can be, but is not limited to, 0.25 mg to 2.0 mg, 0.5 mg to 1.5 mg, 0.75 mg to 1.25 mg, or 1.0 mg. In those aspects of the invention in which trimegestone is administered transdermally or vaginally, the total daily dosage of trimegestone can be, but is not limited to, 0.175 mg to 2.0 mg, 0.35 mg to 1.5 mg, 0.52 mg to 1.25 mg, or 0.7 mg to 1.0 mg.

In further aspects of the invention, the daily dosage of trimegestone in the first and second phases of an ascending-dose extended cycle regimen can be 1.0 mg. In still further embodiments, the daily dosage of trimegestone in the first, second, and third phases of an ascending-dose extended cycle regimen can be 1.0 mg. In those aspects of the invention in which trimegestone is administered transdermally or vaginally, the daily dosage of trimegestone in the first and second phases of art ascending-dose extended cycle regimen can be 0.7 mg to 1.0 mg. In yet other embodiments, the daily dosage of transdermally- or vaginally-administered trimegestone in the first, second, and third phases of an ascending-dose extended cycle regimen can be 0.7 mg to 1.0 mg.

The daily dosage of trimegestone in the first phase can be, but is not limited to, 0.25 mg to 1.25 mg, 0.5 mg to 1.0 mg, or 0.75 mg. The daily dosage of trimegestone in the second phase can be, but is not limited to, 0.5 mg to 1.5 mg, 0.75 mg to 1.25 mg, or 1.0 mg. The daily dosage of trimegestone in the third phase can be, but is not limited to, 0.75 mg to 1.75 mg, 1.0 mg to 1.5 mg, or 1.25 mg.

For those aspects of the invention in which the trimegestone is administered transdermally or vaginally, the daily dosage of trimegestone in the first phase can be, but is not limited to, 0.175 mg to 1.25 mg, 0.35 mg to 1.0 mg, or 0.52 mg to 0.75 mg. The daily dosage of transdermally- or vaginally-administered trimegestone in the second phase can be, but is not limited to, 0.35 mg to 1.5 mg, 0.52 mg to 1.25 mg, or 0.7 mg to 1.0 mg, and the daily dosage in the third phase can be, but is not limited to, 0.52 mg to 1.75 mg, 0.7 mg to 1.5 mg, or 0.87 mg to 1.25 mg.

The trimegestone can be administered together with an estrogen in the ascending-dose extended cycle regimens of the present invention. In addition, the trimegestone can be administered in combination with an estrogen in other oral contraceptive regimens.

For example, the daily dosage of trimegestone for an estrogen and progestin combined 28-day oral contraceptive can be, but is not limited to, 0.25 mg to 2.0 mg, 0.5 mg to 1.75 mg, 0.75 mg to 1.25 mg, or 1.0 mg.

In some aspects of the invention, the daily dosage of estrogen in each of the first and second phases are equal to each other. In further aspects of the invention, the daily dosage of estrogen in each of the first, second, and third phases are equal to each other.

In some aspects of the invention, the daily dosage of estrogen in the second phase is higher than the daily dosage of estrogen in the first phase. In further aspects of the invention, the daily dosage of estrogen in the third phase is higher than the daily dosage of estrogen in the second phase.

In some aspects of the invention, the daily dosage of estrogen in the second phase is equal to the daily dosage of estrogen in the first phase and the daily dosage of estrogen in the third phase is higher than the daily dosage of estrogen in the second phase. In other aspects of the invention, the daily dosage of estrogen in the second phase is higher than the daily dosage of estrogen in the first phase and the daily dosage of estrogen in the third phase is equal to the daily dosage of estrogen in the second phase.

In some aspects of the invention, the daily dosage of estrogen in the second phase is less than twice the daily dosage of estrogen in the first phase. In further aspects of the invention, the daily dosage of estrogen in the third phase is less than twice the daily dosage of estrogen in the second phase.

In some aspects of the invention, the daily dosage of estrogen in the first phase is the equivalent of 15 µg to 25 µg, 17 µg to 23 µg, or 19 µg to 21 µg of ethinyl estradiol. For example, the daily dosage of estrogen in the first phase can be the equivalent of 20 µg of ethinyl estradiol.

In some aspects of the invention, the daily dosage of estrogen in the second phase is the equivalent of 20 µg to 30 µg, 22 µg to 28 µg, or 24 µg to 26 µg of ethinyl estradiol. For example, the daily dosage of estrogen in the second phase can be the equivalent of 25 µg of ethinyl estradiol.

In some aspects of the invention, the daily dosage of estrogen in the third phase is the equivalent of 25 µg to 35 µg, 27 µg to 33 µg, or 29 µg to 31 µg of ethinyl estradiol. For example, the daily dosage of estrogen in the third phase can be the equivalent of 30 µg of ethinyl estradiol.

In some aspects of the invention, the estrogen and progestin are administered orally and the daily dosage of estrogen is the equivalent of 15 µg to 50 µg of ethinyl estradiol and the daily dosage of progestin is the equivalent of 100 µg to 150 µg of levonorgestrel.

In some aspects of the invention, the estrogen and progestin of the ascending-dose extended cycle regimens can be ethinyl estradiol and levonorgestrel, although other suitable estrogens and progestins can be employed. If a different estrogen or progestin is employed, an adjustment in the amount based on the relative potency or activity can be made. Correlations in potency among the various estrogens and among the various progestins are known. See, for example, EP 0 253 607, which is incorporated herein in its entirety by reference. For example, 30 µg of ethinyl estradiol is about the equivalent of 60 µg of mestranol or 2,000 µg of 17β-estradiol. Similarly, 0.050 mg of levonorgestrel is about the equivalent of 0.175 mg of norethindrone acetate, 0.050 mg of desogestrel, 0.050 mg 3-ketodesogestrel, 0.035 mg of gestodene, 0.100 mg of norgestrel, or 0.35-0.50 mg of trimegestone. It should be understood that when norgestrel is used in place of levonorgestrel, its concentration is twice that of levonorgestrel. Norgestrel (dl-norgestrel) is a racemic mixture of optically active isomers, while levonorgestrel is one of the optically active isomers present in norgestrel.

Equivalent concentrations of estrogens and of progestins can be determined using either *in vitro* or *in vivo* assay methods. See, for example, Kuhl, H., Drugs 51(2):188-215 (1996); Philibert, D., et al., Gynecol. Endocrinol. 13:316-326 (1999); and Lundeen, S., et al., J. Steroid Biochem. Molec. Biol. 78:137-143 (2001), in which the relative potencies of various progestins are compared using both *in vitro* and *in vivo* test assays. See also, for example, Dickey, R. P., "Contraceptive Therapy," OBG Management Supplement (October 2000), pp. 2-6. Each of these documents is incorporated herein by reference in its entirety.

For example, various combinations of progestin and estrogen that have been used in oral contraceptives are shown in Table 1.

**TABLE 1**

| Combinations of Progestin and Estrogen | | | | | | |
|---|---|---|---|---|---|---|
| | Norethindrone | | | EE Equivalent | | |
| | Dose | Equivalent | | Dose | Dose | |
| Progestin | (mg) | Dose (mg) | Estrogen | (mg) | (mg) | P/E Ratio |
| Norethynodrel | 9.85 | 9.85 | Mestranol | 0.150 | 0.105 | 93.810 |
| | 5.00 | 5.00 | | 0.075 | 0.053 | 95.238 |
| | 2.50 | 2.50 | | 0.036 | 0.025 | 99.206 |
| | 2.50 | 2.50 | | 0.100 | 0.070 | 35.714 |
| Norethindrone | 10.00 | 10.00 | Mestranol | 0.060 | 0.042 | 238.095 |
| | 2.00 | 2.00 | | 0.100 | 0.070 | 28.571 |
| | 1.00 | 1.00 | | 0.050 | 0.035 | 28.571 |
| | 1.00 | 1.00 | | 0.080 | 0.056 | 17.857 |
| Norethindrone | 1.00 | 1.00 | Ethinyl | 0.050 | 0.050 | 20.000 |
| | 1.00 | 1.00 | estradiol (EE) | 0.035 | 0.035 | 28.571 |
| | 0.50 | 0.50 | | 0.035 | 0.035 | 14.286 |
| | 0.40 | 0.40 | | 0.035 | 0.035 | 11.429 |
| Norethindrone | 2.50 | 2.50 | EE | 0.050 | 0.050 | 50.000 |
| acetate | 1.00 | 1.00 | | 0.050 | 0.050 | 20.000 |
| | 0.60 | 0.60 | | 0.030 | 0.030 | 20.000 |
| | 1.50 | 1.50 | | 0.030 | 0.030 | 50.000 |
| | 1.00 | 1.00 | | 0.020 | 0.020 | 50.000 |
| Ethynodiol | 1.00 | 1.00 | Mestranol | 0.100 | 0.070 | 14.286 |
| diacetate | | | | | | |
| Ethynodiol | 1.00 | 1.00 | EE | 0.050 | 0.050 | 20.000 |
| diacetate | 1.00 | 1.00 | | 0.035 | 0.035 | 28.571 |
| dl-Norgestrel | 0.50 | 0.75 | EE | 0.050 | 0.050 | 10.000 |
| | 0.30 | 0.45 | | 0.030 | 0.030 | 10.000 |
| Levonorgestrel | 0.10 | 0.35 | EE | 0.020 | 0.020 | 5.000 |
| | 0.15 | 0.52 | | 0.030 | 0.030 | 5.000 |

| | | | | | | |
|---|---|---|---|---|---|---|
| Equivalencies 50 mg Mestranol = approx. 35 mg Ethinyl estradiol (EE) 0.1 mg dl-Norgestrel = approx. 0.15 mg Norethindrone | | | | | | |

Each block in Table 1 describes a specific combination of progestin and estrogen, e.g., norethynodrel and mestranol, and within each block older combinations are listed first, with successively newer combinations following.

Suitable progestins for use in the present invention include, but are not limited to, natural and synthetic compounds having progestational activity, such as, for example, progesterone, levonorgestrel, norethindrone, norethindrone acetate, desogestrel, gestodene, dienogest, norgestimate, cyproterone acetate, norelgestromin, etonogestrel, ethynodiol diacetate, norgestrel, trimegestone, medroxyprogesterone acetate, chlormadinone acetate, drospirenone, and other natural and/or synthetic gestagens. Esters, conjugates, and prodrugs of suitable progestins can also be used.

The expression "prodrug" denotes a derivative of a known direct acting drug, which derivative has enhanced delivery characteristics and therapeutic value as compared to the drug and is transformed into the active drug by an enzymatic or chemical process. Ethynodiol diacetate, which is converted *in vivo* to norethindrone, is an example of a progestin prodrug that can be used in the present invention. Additional examples of progestin prodrugs include, but are not limited to, norgestimate (which is converted *in vivo* to 17-deacetyl norgestimate, also known as norelgestromin), desogestrel (which is converted *in vivo* to 3-keto desogestrel, also known as etonogestrel), and norethindrone acetate (which is converted *in vivo* to norethindrone).

Suitable estrogens in the present invention include, but are not limited to, natural and synthetic compounds having estrogenic activity, such as, for example, estradiol (17β-estradiol), 17α-estradiol, estriol, estrone, and their esters, such as the acetate, sulfate, valerate or benzoate esters of these compounds, including, for example, estradiol 17β-cypionate, estradiol 17-propionate, estradiol 3-benzoate, and piperazine estrone sulfate; ethinyl estradiol; conjugated estrogens (natural and synthetic); mestranol; agonistic anti-estrogens; and selective estrogen receptor modulators. Esters, conjugates and prodrugs of suitable estrogens can also be used. Examples of estrogen prodrugs that can be used in the present invention include, but are not limited to, estradiol acetate (which is converted *in vivo* to 17β-estradiol) and mestranol (which is converted *in vivo* to ethinyl estradiol).

In some aspects of the invention, the estrogen and progestin are administered for a period of 31 to 190 consecutive days. Each phase can be, but is not limited to, 7 to 84 days.

In some aspects of the invention, the estrogen and progestin are administered for a period of 39 to 61 days, of 42 to 60 days, of 45 to 57 days, or of 48 to 54 days. For example, the estrogen and progestin can be administered for a period of 49 days to 53 days. The first phase can be, but is not limited to, 7 to 21 days. The second phase can be, but is not limited to, 14 to 28 days. The third phase can be, but is not limited to, 7 to 25 days. In some aspects of the invention, the first phase is 14 days, the second phase is 21 days, and the third phase is 14 days. In other aspects of the invention, the first phase is 14 days, the second phase is 21 days, and the third phase is 18 days.

In some aspects of the invention, the estrogen and progestin are administered for a period of 74 to 96 days, of 77 to 95 days, of 80 to 92 days, or of 83 to 89 days. For example, the estrogen and progestin can be administered for a period of 84 days to 88 days. The first phase can be, but is not limited to, 14 to 28 days. The second phase can be, but is not limited to, 14 to 28 days. The third phase can be, but is not limited to, 35 to 53 days. In some aspects of the invention, the first phase is 21 days, the second phase is 21 days, and the third phase is 42 days. In other aspects of the invention the first phase is 21 days, the second phase is 21 days, and the third phase is 46 days.

In some aspects of the invention, the estrogen and progestin are administered for a period of 95 to 117 days, of 98 to 116 days, of 101 to 113 days, or of 104 to 110 days. For example, the estrogen and progestin can be administered for a period of 105 days to 109 days. The first phase can be, but is not limited to, 14 to 28 days. The second phase can be, but is not limited to, 35 to 49 days. The third phase can be, but is not limited to, 56 to 74 days. In some aspects of the invention, the first phase is 21 days, the second phase is 42 days, and the third phase is 63 days. In other aspects of the invention, the first phase is 21 days, the second phase is 42 days, and the third phase is 67 days.

In some aspects of the invention, the estrogen and progestin are administered for a period of 123 to 145 days, of 126 to 144 days, of 129 to 141 days, or of 132 to 138 days. For example, the estrogen and progestin can be administered for a period of 133 days to 137 days. The first phase can be, but is not limited to, 35 to 49 days. The second phase can be, but is not limited to, 42 to 56 days. The third phase can be, but is not limited to, 35 to 53 days. In some aspects of the invention, the first phase is 42 days, the second phase is 49 days, and the third phase is 42 days. In other aspects of the invention, the first phase is 42 days, the second phase is 49 days, and the third phase is 46 days.

In some aspects of the invention, the estrogen and progestin are administered for a period of 165 to 187 days, of 168 to 186 days, of 171 to 183 days, or of 174 to 180 days. For example, the estrogen and progestin can be administered for a period of 175 days to 179 days. The first phase can be, but is not limited to, 35 to 49 days. The second phase can be, but is not limited to, 56 to 70 days. The third phase can be, but is not limited to, 63 to 81 days. In some aspects of the invention, the first phase is 42 days, the second phase is 63 days, and the third phase is 70 days. In other aspects of the invention, the first phase is 42 days, the second phase is 63 days, and the third phase is 74 days.

In some aspects of the invention, the estrogen and progestin are administered for a period of 180 to 369 days, of 347 to 369 days, of 350 to 366 days, of 353 to 363 days, or of 356 to 362 days. For example, the estrogen and progestin can be administered for a period of 357 days to 361 days. The first phase can be, but is not limited to, 49 to 63 days. The second phase can be, but is not limited to, 168 to 182 days. The third phase can be, but is not limited to, 119 to 137 days. In some aspects of the invention, the first phase is 56 days, the second phase is 175 days, and the third phase is 126 days. In other aspects of the invention, the first phase is 56 days, the second phase is 175 days, and the third phase is 130 days. In some aspects of the invention, the estrogen and progestin are administered in four or more phases.

In some aspects of the invention, the method of providing an ascending-dose extended cycle regimen further includes a hormone-free period. The hormone-free period can be, but is not limited to, 2 to 10 consecutive days. The hormone-free period can be 2 to 8 consecutive days. For example, the hormone-free period can be 3, 5 or 7 days. The hormone-free period can be a non-administration or administration of a placebo. The hormone-free period can include administration of other active ingredients.

In some aspects of the invention, the method of providing an ascending-dose extended cycle regimen further comprises administering estrogen for a period of 2 to 10 consecutive days ("unopposed estrogen interval"). The unopposed estrogen interval can be for a period of 2 to 8 consecutive days. For example, administration of an ascending-dose extended cycle regimen can be followed by administration of the estrogen for a period of 3, 5 or 7 days. The unopposed estrogen interval can include administration of other active ingredients.

Examples of other additional pharmaceutically active ingredients or agents include, but are not limited to, vitamin D or vitamin D analogues; one or more of the B complex vitamins, such as vitamin B3 (niacin (i.e., nicotinic acid and/or nicotinamide)), vitamin B9 (folic acid or folate), vitamin B6 and/or vitamin B12; minerals such as, for example, calcium; iron (e.g., ferrous iron, such as, e.g., ferrous sulfate, ferrous fumarate, ferrous gluconate, or an iron glycine amino acid chelate); agents for preventing and treating bone conditions such as bisphosphonates (e.g., alendronate), teriparatide (e.g., FORTEO™), and SERMs (selective estrogen receptor modulators, e.g., raloxifene).

These additional active agents can be administered during the period of administration of estrogen and progestin, the hormone-free period, the unopposed estrogen interval, or a combination of these periods. For example, vitamin D and/or calcium or a bisphosphonate can be administered during the hormone-free period as a method of maintaining or preventing loss of bone density. Suitable forms of vitamin D and of calcium and bisphosphonate would be known to those of skill in the art. The active ingredients can be provided in the same, different, or separate dosage forms.

In some aspects of the invention, the estrogen that is administered for a period of, e.g., 2 to 10 consecutive days is in a daily dosage that is the equivalent of 5 µg to 50 µg, 5 µg to 30 µg, or 10 µg of ethinyl estradiol.

The ascending-dose extended cycle regimens are optionally administered with an antidepressant. In some aspects of the invention, the antidepressant is administered in combination with estrogen during the unopposed estrogen interval of the regimen. In other aspects of the invention, the antidepressant is administered continuously throughout the regimen, or, in yet other aspects of the invention, the antidepressant is administered intermittently. For example, in some aspects of the invention, the antidepressant is administered intermittently during the late luteal phase, which is typically one to two weeks before menses. In yet other aspects of the invention, the antidepressant is administered one time during a menstrual cycle, or once weekly. For example, in some aspects of the invention, fluoxetine hydrochloride is administered in a one-time or once-weekly dose of about 90 mg. In other aspects of the invention, the antidepressant is administered during the hormone-free period.

The antidepressant that is optionally combined with the ascending-dose extended cycle regimens can be a selective serotonin reuptake inhibitor (SSRI), a selective serotonin and norepinephrine reuptake inhibitor (SSNRI), a tricyclic antidepressant or anxiolytic, or any antidepressant known to one of skill in the art. Suitable antidepressants include, but are not limited to, alprazolam (XANAX^{®}), clomipramine (ANAFRANIL^{®}), fluoxetine (PROZAC^{®}), paroxetine (PAXIL^{®}), sertraline (ZOLOFT^{®}), nefazodone (SERZONE^{®}), fenfluramine (PONDIMIN^{®}) and venlafaxine (EFFEXOR^{®}).

The daily amount of antidepressant administered can vary, depending on the antidepressant used, from about 0.75 to about 2 mg, from about 10 to about 20 mg, or from about 50 to about 100 mg. For example, in some aspects of the invention, fluoxetine hydrochloride is administered in a daily amount of about 5 mg to about 120 mg. A suitable daily amount of antidepressant for administration can be determined by one of skill in the art, e.g., a physician.

Thus, in some aspects of the invention, the method of providing an ascending-dose extended cycle regimen further comprises administering an antidepressant. The antidepressant can be administered (i) during a hormone-free period, (ii) in combination with an estrogen for a period of, e.g., 2 to 10 consecutive days, (iii) continuously, (iv) intermittently, (v) one time, or (vi) once weekly. In some aspects of the invention, the antidepressant is a SSRI such as fluoxetine. In other aspects of the invention, the antidepressant is a SSNRI.

In some aspects of the invention, the administration of an ascending-dose extended cycle regimen of the present invention is followed by monophasic administration of an estrogen and a progestin. As used herein, "monophasic" refers to the continuous use of one particular dose of an estrogen and a progestin during the period of administration of the dosage form of the estrogen and progestin. In some aspects of the invention, the administration of an ascending-dose extended cycle regimen is followed by monophasic administration of an estrogen and a progestin continuously. In some aspects of the invention, the administration of an ascending-dose extended cycle regimen is followed by monophasic administration of an estrogen and a progestin for a period of greater than 30 or 31 consecutive days.

In some aspects of the invention, the administration of an ascending-dose extended cycle regimen is followed by monophasic administration of an estrogen and a progestin for a period of 350 to 370 consecutive days, of 260 to 280 consecutive days, of 175 to 190 consecutive days, or of 60 to 110 consecutive days. The monophasic administration of an estrogen and a progestin can be optionally followed either by a hormone-free period of, e.g., 2 to 10 consecutive days or by administration of estrogen for a period of, e.g., 2 to 10 consecutive days.

### Treatment of Conditions and Disorders

An ascending-dose extended cycle regimen disclosed herein can be used as a method of female contraception. Thus, the invention is directed to a method of contraception in a female in need thereof by administering to the female an ascending-dose extended cycle regimen disclosed herein. The female can be, for example, of childbearing age or peri-menopausal.

However, an ascending-dose extended cycle regimen is also useful as a method of treating a variety of conditions and disorders in females. Thus, an ascending-dose extended cycle regimen can be used as a method of providing contraception to a female for the treatment of a condition or disorder, or as a method of providing contraception and treating a condition or disorder in a female. Such conditions and disorders are described below and include, but are not limited to: breakthrough bleeding; irregular withdrawal bleeding; menstrual bleeding disorders; symptoms associated with an ovarian cyst, uterine leiomyoma (fibroid tumor), and Polycystic Ovarian Syndrome; hirsutism; iron deficiency anemia; menstrual disorders; acne; endometriosis; endometrial cancer; ovarian cancer; benign breast disease; infections; ectopic pregnancy; temporomandibular disorder; catamenial symptoms; non-menstrual related headache, nausea, and depression; peri-menopausal symptoms; hypoestrogenism; menopausal disorders; and loss of bone density.

The invention, therefore, is also directed to a method of providing contraception to a female for the treatment of a condition or disorder, wherein the female is in need of treatment for the condition or disorder, by administering to the female an ascending-dose extended cycle regimen disclosed herein. The female can be, for example, of childbearing age, peri-menopausal, or menopausal.

The invention is also directed to a method of providing contraception and treating a condition or disorder in a female, wherein the female is in need of both contraception and treatment of the condition or disorder, by administering to the female an ascending-dose extended cycle regimen disclosed herein. The female can be, for example, of childbearing age or peri-menopausal.

An ascending-dose extended cycle regimen disclosed herein includes administration to a female beginning at Day 1 of a menstrual cycle that is defined as beginning at the first day of menstrual flow. Alternatively, an ascending-dose extended cycle regimen disclosed herein can also include administration to a female beginning at Day 1 of a menstrual cycle that is defined as beginning with the day after the ending of the menstrual flow. Alternatively, an ascending-dose extended cycle regimen disclosed herein also can include administration to a female beginning at Day 1 of a menstrual cycle that is defmed as beginning with any day within the menstrual cycle.

For each of the disclosed methods of the invention, the effect of administration of an ascending-dose extended cycle regimen, with respect to the specified condition (e.g., inducing the specified condition in the female, reducing the occurrence of the condition, minimizing the condition, or treating the condition or disorder) can be evaluated in comparison to each other, to the condition or disorder exhibited by the female after administration of a conventional or standard 28-day contraceptive regimen, after administration of an extended cycle contraceptive regimen other than an ascending-dose extended cycle regimen of the present invention, and/or with no contraceptive regimen. For example, the effect of administering an ascending-dose extended cycle regimen to treat a menstrual bleeding disorder can be evaluated by comparing the occurrence and/or severity of the bleeding disorder in females suffering from the disorder who have been administered an ascending-dose extended cycle regimen with the occurrence and/or severity of the bleeding disorder in females suffering from the disorder who have not been treated with a contraceptive regimen, or with females suffering from the disorder who have been administered a contraceptive regimen not disclosed in the present invention. The effect of administering an ascending-dose extended cycle regimen of the invention can also be evaluated by comparing the occurrence and/or severity of a condition in a female before and after administration of an ascending-dose extended cycle regimen of the invention, or by evaluating the condition of the female during the course of one or more cycles.

The present invention is directed to a method of reducing breakthrough bleeding in a female in need thereof by administering to the female an ascending-dose extended cycle regimen disclosed herein. The female can be, for example, of childbearing age or peri-menopausal.

The invention is also directed to a method of providing contraception and reducing breakthrough bleeding in a female in need thereof by administering to the female an ascending-dose extended cycle regimen disclosed herein. For example, the female can be of childbearing age or peri-menopausal.

The invention is directed to a method of inducing regular, predictable withdrawal bleeding in a female in need thereof by administering to the female an ascending-dose extended cycle regimen disclosed herein. The female can be, for example, of childbearing age or peri-menopausal. Administration of an ascending-dose extended cycle regimen is useful in controlling menstrual cycles in a female by inducing regular, predictable withdrawal bleeding. By suppressing ovulation and delivering estrogen and progesterone in a programmed fashion, an ascending-dose extended cycle regimen can establish or restore synchrony to the endometrium. This is particularly useful in the treatment of heavy or intermenstrual bleeding. The resulting predictable timing and shorter duration of bleeding are especially advantageous to peri-menopausal women, who often experience irregular menstrual cycles.

The invention is also directed to a method of providing contraception and inducing regular, predictable withdrawal bleeding in a female in need thereof by administering to the female an ascending-dose extended cycle regimen disclosed herein. The female can be, for example, a female of childbearing age or a peri-menopausal female.

The invention is directed to a method of reducing frequency or delaying onset of a menstrual cycle in a female in need of delayed or reduced menstruation by administering to the female an ascending-dose extended cycle regimen disclosed herein. The female can be, for example, of childbearing age or peri-menopausal. For example, particular groups or subpopulations of women can benefit from reduced menstruation, such as women enlisted in the U.S. military and women athletes. Control of the menstrual cycle, or even induction of amenorrhea using an ascending-dose extended cycle regimen, can be an advantage for women on active duty. The non-contraceptive benefits resulting from use of an ascending-dose extended cycle regimen, such as reduction in dysmenorrhea, premenstrual syndrome, menorrhagia, iron deficiency anemia, and ability to control timing of withdrawal bleeding, can be desirable and advantageous to women athletes as well. The term "amenorrhea" refers to the absence of bleeding during one or more menstrual cycles of a female. The term encompasses the absence of bleeding and/or spotting during the unopposed estrogen interval of an ascending-dose extended cycle regimen of the present invention when administered to a female, as well as the absence of bleeding or spotting throughout an entire menstrual cycle during administration of an ascending-dose extended cycle regimen.

The invention is also directed to a method of providing contraception and reducing frequency or delaying onset of a menstrual cycle in a female in need thereof by administering to the female an ascending-dose extended cycle regimen disclosed herein. The female can be, for example, a female of childbearing age or a peri-menopausal female.

The invention is directed to a method for minimizing uterine bleeding in a female in need thereof by administering to the female an ascending-dose extended cycle regimen disclosed herein. The female can be, for example, of childbearing age or peri-menopausal. By diminishing endometrial proliferation, administration of an estrogen and a progestin in an ascending-dose extended cycle regimen can reduce the volume and duration of menstrual flow. A female on a disclosed ascending-dose extended regimen can experience fewer total scheduled days of bleeding than a female on a traditional 28-day regimen, and can experience less blood loss, because an ascending-dose extended cycle regimen involves fewer stop/start transitions per year. The female to be treated can exhibit abnormal uterine bleeding, including, for example, menorrhagia. As used herein, "abnormal uterine bleeding" refers to an abnormal duration of bleeding (i.e., greater than 7 days of bleeding, or hypermenorrhea), abnormal amount of bleeding (i.e., greater than about 80 mL blood loss during menses, or menorrhagia), increased frequency of bleeding (i.e., less than 22 days between menstrual cycles, or polymenorrhea), or any combinations thereof.

The invention is also directed to a method of providing contraception and minimizing uterine bleeding in a female in need thereof by administering to the female an ascending-dose extended cycle regimen disclosed herein. The female can be, for example, a female of childbearing age or a peri-menopausal female.

The invention, moreover, is directed to a method of treating a menstrual bleeding disorder in a female in need thereof by administering to the female an ascending-dose extended cycle regimen disclosed herein. The female can be, for example, of childbearing age or peri-menopausal.

The invention is also directed to a method of providing contraception and treating a menstrual bleeding disorder in a female in need thereof by administering to the female an ascending-dose extended cycle regimen disclosed herein. The female can be, for example, a female of childbearing age or a peri-menopausal female.

The invention is directed to a method of treating symptoms associated with ovarian cysts, uterine leiomyomas (fibroids), or Polycystic Ovarian Syndrome in a female in need thereof by administering to the female an ascending-dose extended cycle regimen disclosed herein. The female can be, for example, of childbearing age, peri-menopausal, or menopausal. The invention is also directed to a method of providing contraception and treating symptoms associated with ovarian cysts, uterine leiomyomas (fibroids), or Polycystic Ovarian Syndrome in a female in need thereof by administering to the female an ascending-dose extended cycle regimen disclosed herein. The female can be, for example, a female of childbearing age or peri-menopausal.

Ovarian cysts, uterine leiomyomas (fibroids), or Polycystic Ovarian Syndrome can cause symptoms including, but not limited to, pelvic pain, dysmenorrhea, abnormal uterine bleeding, acne, and hirsutism. In the invention, such symptoms can be treated by administration of an ascending-dose extended cycle regimen described herein.

Ovarian cysts arise from functional cysts that commonly occur around mid-cycle, when a follicle destined to become an egg fails to mature. Instead of leaving the ovary in a process known as ovulation, it remains inside, floating in a tiny sac of fluid. It is that sac that eventually forms into a cyst. Although rarely malignant, ovarian cysts lead to 200,000 hospitalizations in the United States each year. For some women, some studies have shown that the cysts develop cycle after cycle. Though ovarian cysts can sometimes be asymptomatic, they can also cause pain (constant pelvic pain, pain during intercourse, pain during pelvic movement, and/or pain before or after menses), abnormal bleeding (lengthened, shortened, irregular and/or absent menses), and/or abdominal bloating or distension.

Uterine fibroids are benign growths of uterine muscle that sometimes exist singly, but most often are multiple and range in size from microscopic to filling most of the lower abdominal cavity. Many women with fibroids have no symptoms at all. For those that do, the most common complaints are pressure symptoms and heavy, prolonged periods. There may be pressure in the pelvic region from the enlarged uterus, and the resulting symptoms are often related to where the fibroid is exerting pressure (e.g., increased urinary frequency, constipation or difficulty with bowel movements). The pressure can also cause backache, lower abdominal discomfort, and pain during and after intercourse. Fibroids can cause very heavy and prolonged periods, leading to iron-deficiency anemia, as well as painful periods (secondary dysmenorrhea). The presence of fibroids can also cause reproductive problems such as infertility, multiple miscarriage, premature labor, or labor complications.

The term "ovarian cyst"' as used above represents more singular occurrences caused by the failure of an egg to mature. Polycystic Ovarian Syndrome (PCOS), in contrast, is due to an abnormal production of LH (luteinizing hormone) and FSH (follicle stimulating hormone) by the pituitary gland. An imbalance of these hormones stops egg production and increases production of androgens, with the ovaries producing higher levels of testosterone and estrogens. This results in ovaries "peppered'" with empty egg follicles that become inflamed cysts, irregular or stopped periods (which in turn causes infertility), excess body hair growth, and acne on the face and body. PCOS often leads to obesity, diabetes and hypertension.

Polycystic Ovarian Syndrome is the cause of most cases of androgen-dependent hirsutism. See Rittmaster, R.S., Lancet 349:191-195 (1997). Hirsutism can be described as the growth of excessive hair in women on parts of the body where excessive hair is generally not present, e.g., on the back and chest. Most cases of hirsutism are androgen-dependent, i.e., result from a combination of increased androgen production by the body and increased skin sensitivity to androgens. Normally, small quantities of androgens are produced by the ovaries and the adrenal glands. However, in women suffering from Polycystic Ovarian Syndrome, androgen levels are elevated, which can lead to the development of androgen-dependent conditions such as, for example, pronounced forms of acne (e.g., acne papulopustulosa), androgenetic alopecia and mild forms of hirsutism. Oral contraceptives can suppress the ovarian production of androgens and are thus useful in the treatment of these androgen-dependent conditions.

Thus, the invention is also directed to a method of treating hirsutism in a female in need thereof, by administering to the female an ascending-dose extended cycle regimen disclosed herein. The female can be, for example, of childbearing age, peri-menopausal, or menopausal.

The invention is also directed to a method of providing contraception and treating hirsutism in a female in need thereof, by administering to the female an ascending-dose extended cycle regimen disclosed herein. The female can be, for example, a female of childbearing age or peri-menopausal.

The invention is directed to a method of treating alopecia in a female in need thereof, by administering to the female an ascending-dose extended cycle regimen disclosed herein. The female can be, for example, of childbearing age, peri-menopausal, or menopausal. The invention is also directed to a method of providing contraception and treating alopecia in a female in need thereof, by administering to the female an ascending-dose extended cycle regimen disclosed herein. The female can be, for example, a female of childbearing age or peri-menopausal.

The invention is further directed to a method of decreasing risk of iron deficiency anemia in a female in need thereof by administering to the female an ascending-dose extended cycle regimen disclosed herein. The female can be, for example, of childbearing age or peri-menopausal. A reduction in the volume and duration of menstrual flow that can result from administration of, e.g., an ascending-dose extended cycle regimen can lead to a reduction in the total loss of blood, thus improving the body's iron stores and reducing the morbidity associated with menorrhagia. This effect is particularly desirable in women with coagulation disorders, for example, von Willebrand's disease. The female to be treated can be, but is not limited to, a peri-menopausal female.

The invention is also directed to a method of providing contraception and decreasing risk of iron deficiency anemia in a female in need thereof by administering to the female an ascending-dose extended cycle regimen disclosed herein. The female can be, for example, a female of childbearing age or a peri-menopausal female.

The invention is directed to a method of treating a menstrual disorder in a female in need thereof by administering to the female an ascending-dose extended cycle regimen disclosed herein. The female can be, for example, of childbearing age or peri-menopausal. Menstrual disorders include, but are not limited to, irregular menstrual cycles, dysmenorrhea (painful menstruation), mittelschmerz, and dysfunctional uterine bleeding, as well as premenstrual symptoms such as, but not limited to, those associated with premenstrual syndrome (PMS) or Premenstrual Dysphoric Disorder (PMDD).

The invention is also directed to a method of providing contraception and treating a menstrual disorder in a female in need thereof by administering to the female an ascending-dose extended cycle regimen disclosed herein. The female can be, for example, a female of childbearing age or a peri-menopausal female.

During the luteal phase of the menstrual cycle, as many as 75% of women with regular menstrual cycles experience some symptoms of premenstrual syndrome (PMS), a recurring, cyclical disorder involving behavioral, emotional, social and physical symptoms (Steiner et al., Annu. Rev. Med. 48:447-455 (1997)). Behavioral, emotional and social symptoms include, but are not limited to, irritability, mood swings, depression, hostility and social withdrawal. Physical symptoms include, but are not limited to, bloating, breast tenderness, myalgia, migraines or headaches, and fatigue: True PMS only occurs during the luteal phase of the menstrual cycle, with a symptom-free period during the follicular phase. The etiology of PMS is still unknown.

A subgroup of women with PMS, about 2-9%, exhibit symptoms that are primarily related to a severe mood disorder. In these women, the diagnosis of Premenstrual Dysphoric Disorder (PMDD), which is defined in the Fourth edition of the Diagnostic and Statistical Manual of Mental Disorders (DSM-IV) can be applied. According to the DSM-IV, a woman with PMDD must have at least five premenstrual symptoms during the luteal phase, with at least one of the symptoms being an emotional or "core" symptom. The core symptoms must be irritability, anger, mood swings, tension or depression (and interfere with daily activities), and must be confirmed by a prospective daily rating for at least two cycles. Three to five percent of women with PMS report to have PMDD. There is also a subgroup of women who experience severe PMS, which accounts for about 20% of the PMS population. These women experience severe emotional symptoms that do not fall under the strict criteria of PMDD as defined in DSM-IV but require medical attention. U.S. Appl. No. 10/309,313 relates to the use of estrogen/progestin contraceptive regimens optionally combined with an antidepressant for the treatment of PMS, PMDD, and related conditions.

Suppression of ovulation that can result from administration of the extended cycle regimen can also eliminate mid-cycle pain ("mittelschmerz") associated with rupture of the ovarian follicle. Additionally, suppression of ovulation and delivery of estrogen and progesterone in a regular, predictable schedule, which can result from use of an ascending-dose extended cycle regimen can be beneficial in the treatment of other menstrual disorders such as heavy or intermenstrual bleeding. In some aspects of the invention, the female can be a peri-menopausal female.

The invention is directed to a method of treating acne in a female in need thereof by administering to the female an ascending-dose extended cycle regimen disclosed herein. The female can be, for example, of childbearing age, peri-menopausal, or menopausal. An ascending-dose extended cycle regimen may suppress gonadotropin and decrease ovarian and adrenal androgen production, resulting in an improvement in acne of, e.g., women of childbearing age and older.

The invention is also directed to a method of providing contraception and treating acne in a female in need thereof by administering to the female an ascending-dose extended cycle regimen disclosed herein. The female can be, for example, a female of childbearing age or peri-menopausal.

The invention is directed to a method of treating endometriosis in a female in need thereof by administering to the female an ascending-dose extended cycle regimen disclosed herein. The female can be, for example, of childbearing age or peri-menopausal. The invention is also directed to a method of providing contraception and treating endometriosis in a female in need thereof by administering to the female an ascending-dose extended cycle regimen disclosed herein. The female can be, for example, a female of childbearing age or a peri-menopausal female.

In hormonal therapy of endometriosis, endometriotc tissue responds to adverse endocrine environments (low estrogen and/or high progestin concentration). Progestins produce marked atrophy of the endometrium and ectopic endometrial tissue and decrease intraperitoneal inflammation associated with endometriosis. The American College of Obstetrics and Gynecology stated that progestins, alone or in combination with estrogens as oral contraceptives, are an optimal choice for the management of endometriosis in women who desire contraception (American College of Obstetricians and Gynecologists, *ACOG Practice Bulletin No. 11* (December 1999)). The use of an ascending-dose extended cycle regimen of the present invention can be beneficial for treating or preventing endometriosis.

Chronic pelvic pain often precedes and is associated with the development of endometriosis. Thus, the invention is also directed to a method of treating chronic pelvic pain in a female in need thereof by administering to the female an ascending-dose extended cycle regimen disclosed herein. The female can be, for example, of childbearing age or peri-menopausal. The invention is also directed to a method of providing contraception and treating chronic pelvic pain in a female in need thereof by administering to the female an ascending-dose extended cycle regimen disclosed herein. The female can be, for example, a female of childbearing age or a peri-menopausal female.

The invention is further directed to a method of reducing the risk of endometrial cancer in a female in need thereof by administering to the female an ascending-dose extended cycle regimen, disclosed herein. The female can be, for example, of childbearing age, peri-menopausal, or menopausal. The invention is also directed to a method of providing contraception and reducing the risk of endometrial cancer in a female in need thereof by administering to the female an ascending-dose extended cycle regimen disclosed herein. The female can be, for example, a female of childbearing age or peri-menopausal.

The invention is directed to a method of reducing the risk of ovarian cancer in a female in need thereof by administering to the female an ascending-dose extended cycle regimen disclosed herein. The female can be, for example, of childbearing age, peri-menopausal, or menopausal. The frequency of ovulation and thereby the frequency of ovarian stimulation can be reduced, suppressed, or eliminated by use of an ascending-dose extended cycle regimen. The invention is also directed to a method of providing contraception and reducing the risk of ovarian cancer in a female in need thereof by administering to the female an ascending-dose extended cycle regimen disclosed herein. The female can be, for example, a female of childbearing age, peri-menopausal, or menopausal.

The invention is further directed to a method of treating benign breast disease, including, but not limited to, fibrocystic breast disease, in a female in need thereof by administering to the female an ascending-dose extended cycle regimen disclosed herein. The female can be, for example, of childbearing age, peri-menopausal, or menopausal. About a third of all women between the ages of 30 and 50 will be diagnosed with fibrocystic breast disease or other benign breast condition. Other terms for this condition include chronic mastitis (inflammation) and mammary dysplasia.

The invention is also directed to a method of providing contraception and treating benign breast disease in a female in need thereof by administering to the female an ascending-dose extended cycle regimen disclosed herein. The female can be, for example, a female of childbearing age or peri-menopausal.

The invention is also directed to a method of reducing the risk of colorectal cancer in a female in need thereof by administering to the female an ascending-dose extended cycle regimen disclosed herein. The female can be, for example, of childbearing age, peri-menopausal, or menopausal. An ascending-dose extended cycle regimen of the present invention is thought to protect against colorectal cancer as a result of changes in bile synthesis and secretion due to the female hormones in the regimen, which is thought to lead to a reduced concentration of bile acids in the colon. It has also been observed that estrogen inhibits the growth of colon cancer cells in vitro, and estrogen receptors have been identified in normal and neoplastic colon epithelial cells. See Fernandez, E., et al., British J. Cancer 84:722-727 (2001). Thus, an ascending-dose extended regimen can be beneficial in the prevention or reduction in the occurrence of colorectal cancer.

The invention is also directed to a method of providing contraception and reducing the risk of colorectal cancer in a female in need thereof by administering to the female an ascending-dose extended cycle regimen disclosed herein. The female can be, for example, a female of childbearing age or peri-menopausal.

The invention is directed to a method of preventing or treating an infection in a female in need thereof by administering to the female an ascending-dose extended cycle regimen disclosed herein. The female can be, for example, of childbearing age, peri-menopausal, or menopausal. For example, sexually transmitted diseases (STDs) are infections caused by a pathogen such as a virus, bacterium, parasite, or fungus, that is spread from person to person through sexual contact. STDs can be painful, irritating, and even life-threatening. An ascending-dose extended cycle regimen is believed to have a protective role against the development of some STDs because it stimulates the body to produce a thicker cervical mucous, which acts as a barrier to semen carrying bacteria that cause sexually transmitted diseases.

The invention is also directed to a method of providing contraception and treating an infection in a female in need thereof by administering to the female an ascending-dose extended cycle regimen disclosed herein. The female can be, but is not limited to, a female of childbearing age or peri-menopausal.

Pelvic Inflammatory Disease (PID) is a complication that can result from STD infections. PID is a serious syndrome of the female reproductive tract that results from the spread of infections (most often sexually transmitted infections such as *Chlamydia trachomatis* and *Nisseris gonnorrheoea*) from the vagina and endocervix to the uterus, fallopian tubes and ovaries. PID is commonly manifested as endometritis (infection of the lining of the uterus) or salpingitis (infection of the fallopian tubes), and less commonly as pelvic peritonitis and/or inflammation of contiguous structures. (MacDonald, N.E., and Bowie, W.R., Canadian Communicable Disease Report 21S4: 25-33 (1995); Westrom, L. and Mardh, P-A., Sexually Transmitted Diseases, 2nd Ed., pages 593-613, New York: McGraw-Hill, 1990).

PID is a major cause of infertility and ectopic pregnancy. Ectopic pregnancy results from the implantation of a fertilized ovum in the fallopian tube or in the abdominal cavity and is thought to be caused by ciliary dysfunction within the fallopian tube resulting from prior tubal infection with *N. gonorrhoea* and/or *C trachomatis,* which often results in loss of ciliated epithelial cells from the fallopian tubes. It has been estimated that prior tubal infection with STD agents causes about 50% of the cases of ectopic pregnancy. (MacDonald, N.E., and Brunham, R., Canadian Journal of Human Sexuality 6(2):161-170 (1997).)

An ascending-dose extended cycle regimen is believed to have a protective role against the development of PID because it stimulates the body to produce thicker cervical mucous, which helps prevent semen carrying STD-causing bacteria from gaining access to the uterus and eventually causing PID and PID-related complications, such as ectopic pregnancy.

Thus, an ascending-dose extended cycle regimen of the present invention can be useful in the prevention or reduction in occurrence of sexually transmitted diseases, Pelvic Inflammatory Disease, and ectopic pregnancy. Accordingly, the invention is directed to a method of preventing or reducing the occurrence of a sexually transmitted disease or Pelvic Inflammatory Disease in a female in need thereof by administering to the female an ascending-dose extended cycle regimen disclosed herein. The invention is also directed to a method of preventing ectopic pregnancy in a female in need thereof by administering to the female an ascending-dose extended cycle regimen disclosed herein. The female can be, but is not limited to, a female of childbearing age or a peri-menopausal female.

The invention is also directed to a method of providing contraception and treating a sexually transmitted disease or Pelvic Inflammatory Disease in a female in need thereof by administering to the female an ascending-dose extended cycle regimen disclosed herein. The invention, moreover, is directed to a method of providing contraception and preventing ectopic pregnancy in a female in need thereof by administering to the female an ascending-dose extended cycle regimen disclosed herein. The female can be, but is not limited to, a female of childbearing age or a peri-menopausal female.

In addition, use of an ascending-dose extended cycle regimen, in comparison to the use of a conventional 28-day contraceptive regimen, can lead to a reduction in the reported occurrences of infection such as urinary tract infections, pharyngitis, upper respiratory tract infections, and sinusitus. Thus, the invention is further directed to the prevention or reduction in occurrence of certain infections, such as urinary tract infections, pharyngitis, upper respiratory tract infections, and sinusitus, in a female in need thereof by administering to the female an ascending-dose extended cycle regimen disclosed herein. The female can be, for example, of childbearing age, peri-menopausal, or menopausal. The invention is also directed to a method of providing contraception and treating certain infections, such as urinary tract infections, pharyngitis, upper respiratory tract infections, and sinusitus, in a female in need thereof by administering to the female an ascending-dose extended cycle regimen disclosed herein. The female can be, for example, of childbearing age or peri-menopausal.

The invention is also directed to a method of treating temporomandibular disorder in a female in need thereof by administering to the female an ascending-dose extended cycle regimen disclosed herein. The female can be, for example, of childbearing age, peri-menopausal, or menopausal. Temporomandibular disorders (TMD) are disorders of the jaw muscles, temporomandibular joints, and/or the nerves associated with chronic facial pain. An ascending-dose extended cycle regimen of the present invention can be useful in the treatment of TMD. The invention is also directed to a method of providing contraception and treating temporomandibular disorder in a female in need thereof by administering to the female an ascending-dose extended cycle regimen disclosed herein. The female can be, for example, of childbearing age or peri-menopausal.

The invention is directed to a method of treating a catamenial symptom in a female in need thereof by administering to the female an ascending-dose extended cycle regimen disclosed herein. The female can be, for example, of childbearing age or peri-menopausal. Catamenial symptoms are those associated with conditions, disorders, or diseases that can worsen around the time of menses. Such conditions, disorders, or diseases include, but are not limited to, asthma, rheumatoid arthritis, migraine headaches, seizure disorders or epilepsy, multiple sclerosis, and diabetes. The invention is also directed to a method of providing contraception and treating a catamenial symptom in a female in need thereof by administering to the female an ascending-dose extended cycle regimen disclosed herein. The female can be, for example, of childbearing age or peri-menopausal.

Arthritis is a prevalent chronic condition in women. Hormonal factors can influence the frequency and severity of arthritis. In some women, arthritis symptoms such as joint stiffness, swelling and pain peak during the postovulatory phase of the menstrual cycle, and cyclic changes in local antibody release, white blood cell subpopulations and altered pain perception have been proposed as possible mechanism (Case, A.M. and Reid, R.L., Arch. Intern. Med. 158:1405-1412 (1998)). Estrogen administered as a single agent, and as part of a combined oral contraceptive has been reported to benefit some women (Kay, C.R and Wingrave, S.J., Lancet 1:1437 (1983); Linos, A., et al., Lancet 1:1871 (1978)). Thus, use of an ascending-dose extended cycle regimen can be beneficial as a method of treating a catamenial symptom, such as, e.g., a symptom associated with rheumatoid arthritis, in a female in need thereof.

Approximately 60% of women with migraines report a relationship to menstruation (Case, A.M. and Reid, R.L., Arch. Intern. Med. 158:1405-1412 (1998)). Decreasing levels of estrogen during the late luteal phase of the menstrual cycle or abrupt withdrawal of estrogen as during the non-administration period in women taking oral contraceptives are thought to trigger migraine attacks (Sulak P.J., et al., Obstet. Gynecol 95:261-266 (2000); Kudrow, L., Headache 15:36-49 (1975); Whitty, C.W.M., et al., Lancet 1:856-859 (1966)). Thus, use of an ascending-dose extended cycle regimen can be beneficial as a method of treating a catamenial symptom in a female in need thereof, such as, e.g., a migraine headache in a female.

Catamenial epilepsy refers to seizure disorders that occur or worsen around menstruation. It is believed to result from cyclic alterations in both ovarian hormone levels and drug metabolism (Case, A.M. and Reid, R.L., Arch. Intern. Med. 158:1405-1412 (1998)). Thus, use of an ascending-dose extended cycle regimen can be beneficial as a method of treating a catamenial symptom such as, e.g., a symptom associated with epilepsy, in a female in need thereof

The invention is directed to a method of treating headache or nausea unrelated to the menstrual cycle in a female in need thereof by administering to the female an ascending-dose extended cycle regimen disclosed herein. The female can be, for example, of childbearing age, peri-menopausal, or menopausal. Use of an ascending-dose extended cycle regimen, in comparison to the use of a conventional 28-day contraceptive regimen, can lead to a reduction in the reported occurrences of non-menstrual-related headache and nausea. Thus, a disclosed ascending-dose extended cycle regimen can be used as a method of preventing or treating non-menstrual-related headache and nausea. The invention is also directed to a method of providing contraception and treating headache or nausea unrelated to the menstrual cycle in a female in need thereof by administering to the female an ascending-dose extended cycle regimen disclosed herein. The female can be, for example, of childbearing age or peri-menopausal.

The invention is directed further to a method of treating depression unrelated to the menstrual cycle in a female in need thereof by administering to the female an ascending-dose extended cycle regimen disclosed herein. The female can be, for example, of childbearing age, peri-menopausal, or menopausal. "Depression" is a term that is often used to refer to different forms of depressive disorders and includes major depression, bipolar disorder (sometimes called manic-depressive illness), and dysthymia, a less severe form of depression. Major depression is manifested by a combination of symptoms that interfere with the ability to work, study, sleep, eat and enjoy once pleasurable activities. Bipolar disorder, which is not nearly as prevalent as other forms of depressive disorders, is characterized by cycling mood changes. Dysthymia, a less severe type of depression, involves long-term, chronic symptoms that do not disable, but keep one from functioning well or from feeling well. "Depression" also includes the less severe, temporary sadness and loneliness often felt from time to time. Use of an ascending-dose extended cycle regimen, compared to use of a conventional 28-day contraceptive regimen, can lead to a reduction in the reported occurrences of non-menstrual-related depression. Thus, a disclosed ascending-dose extended regimen can be used as a method of preventing or treating non-menstrual-related depression.

The invention is also directed to a method of providing contraception and treating depression unrelated to the menstrual cycle in a female in need thereof by administering to the female an ascending-dose extended cycle regimen disclosed herein. The female can be, for example, of childbearing age or peri-menopausal.

The invention is further directed to a method of increasing contraceptive effectiveness in a female in need thereof by administering to the female an ascending-dose extended cycle regimen disclosed herein. The female can be, but is not limited to, a female of childbearing age or a peri-menopausal female. A female in need of contraceptive effectiveness can be, but is not limited to, a higher weight female. A "higher weight female" refers to a human female weighing about 70 kg or more or having a body mass index (BMI) of greater than about 25. In a recent study of body weight and oral contraceptive failure, women weighing about 70.5 kg or more were reported to have a 60% higher risk of oral contraceptive failure (Holt, V.L., et al., Obstet. Gynecol. 99:820-827 (2002)).

Thus, the invention is directed to a method of increasing contraceptive effectiveness in a higher-weight female in need thereof by administering to the female an ascending-dose extended cycle regimen disclosed herein. The invention is directed to a method of increasing the contraceptive effectiveness in a human female weighing about 70 kg or more, weighing about 80 kg or more, or weighing about 90 kg or more, by administering to the female an ascending-dose extended cycle regimen.

A disclosed ascending-dose extended cycle regimen can also be used as a method of increasing the contraceptive effectiveness in a human female with a body mass index of greater than about 25, greater than about 30, or greater than about 35. Thus, the invention is also directed to a method of increasing the contraceptive effectiveness in a human female with a body mass index of greater than about 25, greater than about 30, or greater than about 35, by administering to the female an ascending-dose extended cycle regimen.

The invention is also directed to a method of increasing fertility in a female in need thereof, by administering to the female an ascending-dose extended cycle regimen disclosed herein, followed by discontinuation of the regimen and optional administration of an agent to induce ovulation in the female. The female can be, but is not limited to, a female of childbearing age or a peri-menopausal female.

It has been observed clinically that women who are taking oral contraceptives for anovulation often conceive when pills are missed, or shortly after discontinuing oral contraceptive treatment, most likely due to a "rebound effect" occurring in the ovary at least for a short period of time. Suppression of ovarian activity using oral contraceptive pills for 2-6 months may result in decreases in early follicular ovarian androgen production and LH and estradiol levels. Increased androgen levels have been shown to have adverse effects on folliculogenesis. These endocrine changes in the early follicular phase may be responsible for improved ovarian response to clomiphene or other treatments for anovulatory infertility. See Brannigan, E.F., and Estes, M.A., Am. J. Obstet. Gynecol. 188:1424-1430 (2003).

Examples of agents that induce ovulation, and that can be administered following discontinuation of an ascending-dose extended cycle regimen of the present invention, include, but are not limited to, menotropins (Follicle Stimulating Hormone (FSH) and Luteinizing Hormone (LH), e.g., Pergonal®) and chlomiphene citrate (Clomid®). The ovulation-inducing agent can be administered during a suitable time as can be determined by one of skill in the art, e.g., a physician. In some aspects of the invention, the ovulation-inducing agent can be administered, e.g., within about one week to about one month, or within about one week to about two weeks, after discontinuation of an ascending-dose extended cycle regimen of the present invention. In some aspects of the invention, the ovulation-inducing agent is administered, e.g., 2 to 10 days, or 5 to 9 days after discontinuation of an ascending-dose extended cycle regimen.

Thus, the invention is directed to a method of increasing fertility in a female in need thereof, the method comprising (i) administration to the female of an ascending-dose extended cycle regimen disclosed herein; (ii) discontinuation of administration of an ascending-dose extended cycle regimen; and (iii) optional administration to the female of an ovulation-inducing agent during the discontinuation of administration of an ascending-dose extended cycle regimen; wherein fertility in the female is increased.

In some aspects of the invention, the disclosed methods are particularly useful in peri-menopausal women and/or menopausal women. Peri-menopausal and menopausal women frequently experience a large variety of conditions and disorders that have been attributed to estrogen deprivation due to ovarian failure or hypoestrogenism. The duration of these disorders can be extremely variable and include hot flushes which can be devastating in some women and very mild in others. Dryness of the vagina associated with susceptibility to minor infections, and frequently associated with discomfort during intercourse, is another symptom that can be directly related to the decrease in estrogen availability.

In a long-term sense, one of the most health-threatening aspects of menopause is the loss of mineral from bone which can result in a decrease in bone mass (osteoporosis) and generates a serious risk of fractures. For example, evidence exists that there is a six-fold increase in fractures in postmenopausal women as opposed to men of the same age (Garraway et al., Mayo Clinic Proceedings 54:701-707 (1979)). These fractures, of course, carry a high complication rate among older people, a marked increase in disability and general morbidity, and certainly an increased risk of mortality.

Another serious health-threatening aspect of menopause is the impressive loss of protection against heart attacks, which is enjoyed by younger women up to the age of 60, when compared to men of the same age. The steep increase in mean serum cholesterol concentration, which occurs around menopause (during the fourth and fifth decades), can contribute importantly to the progressive increase in death from ischemic heart disease in older women. In the eighth and ninth decades, the incidence of deaths from ischemic heart disease, approaches that of men (Havlik, R.J. and Manning-Feinleid, P.H., NIH Publication No. 79-1610, U.S. Department of HEW (1979)).

Accordingly, the invention is directed to a method for treating conditions, such as the physical conditions described above, resulting from menopausal estrogen decline in a menopausal female by administering an ascending-dose extended cycle regimen disclosed herein to the female. The invention is also directed to a method for treating conditions, such as the physical conditions described above, resulting from hypoestrogenism in a female by administering an ascending-dose extended cycle regimen disclosed herein to the female. The invention is further directed to a method for treating conditions, such as the physical conditions described above, resulting from ovarian failure in a female by administering an ascending-dose extended cycle regimen disclosed herein to the female.

The invention is also directed to a method of providing contraception and treating conditions, such as the physical conditions described above, resulting from hypoestrogenism in a peri-menopausal female in need thereof by administering an ascending-dose extended cycle regimen disclosed herein to the peri-menopausal female. The invention is further directed to a method of providing contraception and treating conditions, such as the physical conditions described above, resulting from ovarian failure in a peri-menopausal female in need thereof by administering an ascending-dose extended cycle regimen disclosed herein to the peri-menopausal female.

In addition to the above-mentioned major physical problems, some menopausal and peri-menopausal women experience a large variety of other symptoms ranging from depression, insomnia, and nervousness, to symptoms of arthritis and so forth.

It is generally agreed that estrogen is the most effective agent for the control or prevention of menopausal flushes and vaginal atrophy. It is effective in retarding or preventing the appearance of clinical evidence of osteoporosis. In appropriate doses, when combined with progestin, a favorable effect upon blood lipids can also be seen. Problems with estrogen therapy do exist, however, and have been widely explored and documented in the medical literature. The means by which estrogen has been administered, generally speaking, involves either the use of estrogen alone or estrogen plus a progestin.

Estrogen alone, given in small doses on a continuous basis, is effective in most patients for the control of the above symptoms and problems associated therewith. However, although the vast majority of women taking continuous low-dose estrogen will not have bleeding for many months or even years, there is a distinct risk posed by this routine of silently (i.e., exhibiting no overt symptoms) developing "hyperplasia of the endometrium." This term refers, of course, to an overstimulation of the lining of the uterus which can become pre-malignant, coupled with the possibility that the patient will eventually develop cancer of the uterine lining even under such a low-dose regimen (Gusberg et al., Obstetrics and Gynaecology 17:397-412 (1961)).

Estrogen alone can also be given in cycles, usually 21-25 days on treatment and 5-7 days off treatment. Again, if small doses of estrogen are required to control the symptoms and it is used to this fashion, only about 10% of women will experience withdrawal bleeding between the cycles of actual treatment. However, one must again be concerned by the risk of developing endometrial hyperplasia and by the increased relative risk of developing cancer of the uterus (Research on the Menopause: Report of a W.H.O. Scientific Group, 53-68 (1981)).

The addition of progestin with estrogen, however, as in an ascending-dose extended cycle disclosed herein, will virtually eliminate the concern about developing endometrial hyperplasia and reduce the risk of developing endometrial carcinoma below that of the untreated general population.

Thus, the invention is directed to a method of treating a menopausal disorder or a peri-menopausal disorder or symptom in a female in need thereof by administering to the female an ascending-dose extended cycle regimen disclosed herein. The invention is also directed to a method of providing contraception and treating a peri-menopausal disorder or symptom in a peri-menopausal female in need thereof by administering to the female an ascending-dose extended cycle regimen disclosed herein.

An ascending-dose extended cycle regimen can be used as a method of maintaining bone density or preventing loss of bone density in a female. An ascending-dose extended cycle regimen can also be used in this way by administering calcium and/or vitamin D, e.g., in combination with the administration of an estrogen and a progestin.

An ascending-dose extended cycle regimen is not limited to administration to peri-menopausal or menopausal females as a method of maintaining bone density or preventing bone loss. An ascending-dose extended cycle regimen can also be used in a method of maintaining bone density or preventing bone loss by administration to a female of childbearing age that is not peri-menopausal or menopausal. For example, an ascending-dose extended cycle regimen can be used with females 12-16 years of age who have not yet achieved peak bone density, but who, due to various conditions such as anorexia, are at risk of loss of bone density or at risk of not achieving a normal physiologic bone density for age and developmental maturity.

Thus, an ascending-dose extended cycle regimen can also be used as a method of treating a condition resulting from menopausal or peri-menopausal estrogen decline, including osteoporosis. An ascending-dose extended cycle regimen can also be used as a method of providing contraception and treating a condition in a peri-menopausal female in need thereof resulting from peri-menopausal estrogen decline, including osteoporosis.

An ascending-dose extended cycle regimen can also be used as a method of treating a female in need of hormone replacement therapy.

### Modes of Administration and Compositions

The estrogen and/or progestin are administered in the conventional manner by any route where they are active. For example, administration can be by, but is not limited to, oral, parenteral, subcutaneous, intravenous, intramuscular, intraperitoneal, transdermal, buccal, or ocular routes, or intravaginally, by inhalation, by depot injections, or by hormone implants. Thus, the dosage forms for the estrogen and/or progestin (either alone or in combination with other pharmaceuticals) can be, but are not limited to, sublingual, injectable (including short-acting, depot, implant and pellet forms injected subcutaneously or intramuscularly), vaginal creams, suppositories, pessaries, rings, rectal suppositories, intrauterine devices, and transdermal forms such as patches and creams.

Thus, pharmaceutical compositions containing the estrogen and/or progestin and a suitable carrier can be solid dosage forms which include, but are not limited to, tablets, capsules, cachets, pellets, pills, powders and granules; topical dosage forms which include, but are not limited to, solutions, powders, fluid emulsions, fluid suspensions, semi-solids, ointments, pastes, creams, gels and jellies, and foams; and parenteral dosage forms which include, but are not limited to, solutions, suspensions, emulsions, and dry powder. It is known in the art that the active ingredients can be contained in such compositions with pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. The means and methods for administration are known in the art and an artisan can refer to various pharmacologic references for guidance. For example, "Modern Pharmaceutics", Banker & Rhodes, Marcel Dekker, Inc. 1979; and "Goodman & Gilman's The Pharmaceutical Basis of Therapeutics," 6th Edition, MacMillan Publishing Co., New York 1980 can be consulted.

For oral administration, the estrogen and/or progestin can be formulated readily by combining these compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol and sorbitol; cellulose preparations such as, but not limited to, maize starch, wheat starch, rice starch, potato starch, gelatin, gum tragacanth, methyl cellulose, hydroxypropylmethyl-cellulose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

The pharmaceutical compositions of the estrogen and/or progestin also can comprise suitable solid or gel phase carriers or excipients such as calcium carbonate, calcium phosphate, various sugars, starches, cellulose derivatives, gelatin, and polymers such as, e.g., polyethylene glycols.

Dragee cores can be provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally include, but are not limited to, push-fit capsules made of gelatin, as well as soft, sealed capsules made of gelatin and a plasticizer, such as glycerol or sorbitol. The push-fit capsules can contain the active ingredients in admixture with filler such as, e.g., lactose, binders such as, e.g., starches, and/or lubricants such as, e.g., talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All compositions for oral administration should be in dosages suitable for such administration.

For buccal administration, the estrogen and progestin compositions can take the form of tablets or lozenges formulated in conventional manner.

For administration by inhalation, the estrogen and/or progestin for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The estrogen and/or progestin can be formulated for parenteral administration by injection, e.g., by bolus injection or continuous infusion. The compounds can be administered by continuous infusion subcutaneously over a period of about 15 minutes to about 24 hours. Compositions for injection can be presented in unit dosage form, e.g., in ampoules or in multidose containers, with an added preservative. The compositions can take such forms as suspensions, solutions or emulsions in oily or aqueous vehicles, and can contain formulatory agents such as suspending, stabilizing and/or dispersing agents.

The estrogen and/or progestin can also be formulated in rectal compositions such as suppositories or retention enemas, e.g., containing conventional suppository bases such as cocoa butter or other glycerides.

In addition to the compositions described previously, the estrogen and/or progestin can also be formulated as a depot preparation. Such long acting compositions can be administered by implantation (e.g., subcutaneously or intramuscularly) or by intramuscular injection. Depot injections can be administered at about 1 to about 6 months or longer intervals. Thus, for example, the estrogen and/or progestin can be formulated with suitable polymeric or hydrophobic materials (e.g., as an emulsion in an acceptable oil) or ion exchange resins, or as sparingly soluble derivatives, for example, as a sparingly soluble salt.

For transdermal administration, the estrogen and/or progestin can be applied by any transdermal, therapeutic system that is consequently supplied to the organism, such as, for example, as a transdermal patch, transdermal cream or plaster. For example, the estrogen and/or progestin can be formulated as a transdermal patch. The preparation and use of transdermal patches are well known to those of skill in the art and are available in different designs, including matrix-type or reservoir-type designs. In addition to the estrogen and/or progestin, transdermal patches can contain additional components such as penetration-enhancing agents and/or additional excipients that are conventionally employed, such as, e.g., carriers, gelling agents, suspending agents, dispersing agents, preservatives, stabilizers, wetting agents, emulsifying agents, and the like.

For vaginal administration, the estrogen and/or progestin can be formulated as vaginal gels, creams, tampons, suppositories, vaginal rings, intrauterine devices and the like. The preparation of each of these formulations is well known to those of skill in the art.

The estrogen and/or progestin can also be administered with other active ingredients. The hormone-free period or the unopposed estrogen interval can also include administration of other active ingredients. For example, as described above, estrogen and/or progestin can be administered in combination with an antidepressant. Estrogen and/or progestin can also be administered with vitamin D and/or calcium in the ascending-dose extended cycle regimens as a method of maintaining or preventing loss of bone density. Alternatively, vitamin D and/or calcium can be administered in the ascending-dose extended cycle regimens during the unopposed estrogen interval following administration of estrogen and/or progestin. The form of vitamin D and of calcium used in the present invention would be well known to those of skill in the art, as would the amount. For example, calcium can be administered in the form of calcium carbonate, at a dosage level of e.g., 500 mg.

In some aspects of the invention, the estrogen and/or progestin are in a oral, transdermal, intravaginal, implantable pellet, or injectable liquid dosage form. For example, the estrogen and/or progestin can be in an oral dosage form, a transdermal dosage form, or an intravaginal dosage form.

In some aspects of the invention, the estrogen and progestin can be combined in one dosage form. In other aspects of the invention, the estrogen and progestin can be provided in different or separate dosage forms. Similarly, other active ingredients can be provided in the same, different, or separate dosage forms.

In some aspects of the invention, each phase of the regimen of the invention can be administered in a separate, single dosage form. In other aspects of the invention, each phase of the regimen of the invention can be administered in one or more separate dosage forms. For example, each phase can be administered using a separate ring or transdermal device (such as a patch). As another example, each phase can be administered using one or more rings or transdermal devices.

The present invention also provides a combination of two or more modes of administration with each regimen. For example, the estrogen can be provided by transdermal administration and the progestin can be provided by vaginal administration. As another example, the estrogen can be provided by transdermal administration, the progestin can be provided by transdermal administration, and an SSRI can be provided by oral administration.

### Kits and Patient Education

The dosages or compositions for the ascending-dose extended cycle regimens of the invention can be provided in the form of a kit or package, with the dosages arranged for proper sequential administration. For example, in the oral form of the composition, the present invention provides a pharmaceutical package which contains combination-type contraceptives in multiple dosage units in a synchronized, fixed sequence, wherein the sequence or arrangement of the dosage units corresponds to the stages of daily administration.

Thus, for example, the pharmaceutical compositions useful in the invention can be provided in kit form containing greater than 30 or 31 tablets intended for ingestion on successive days. In some embodiments, the kit further contains, e.g., 2 to 10 tablets, intended for ingestion on successive days following the ingestion of the greater than 30 or 31 tablets. Administration is daily for a period of greater than 30 or 31 consecutive days using tablets containing both the estrogen and the progestin, and, in some embodiments, is followed by administration that is daily for, e.g., 2 to 10 consecutive days using either placebo tablets or tablets containing estrogen. For example, administration can be for 40-190 consecutive days, using tablets containing both estrogen and the progestin, followed by administration for, e.g., at least 2-10 days with estrogen, using tablets containing estrogen. As another example, administration can be for 75-95 days, using tablets containing both an estrogen and a progestin, followed by administration for, e.g., at least 2-10 days with estrogen, using tablets containing estrogen. As yet another example, administration can be for 168-186 days, using tablets containing both an estrogen and a progestin, followed by administration for, e.g., at least 2-10 days with estrogen, using tablets containing estrogen.

Some aspects of the invention provide a pharmaceutical kit comprising a first vaginal ring capable of providing a daily dosage of estrogen and a daily dosage of progestin; a second vaginal ring capable of providing a daily dosage of estrogen that is equal to or higher than that of the first vaginal ring and capable of providing a total daily dosage of estrogen and progestin that is higher than that of the first vaginal ring; and a third vaginal ring capable of providing a daily dosage of estrogen that is equal to or higher than that of the second vaginal ring and capable of providing a total daily dosage of estrogen and progestin that is higher than that of the second vaginal ring; wherein the vaginal rings are capable of providing estrogen and progestin for a period of greater than 30 or 31 consecutive days.

Other aspects of the invention provide a pharmaceutical kit comprising a first vaginal ring capable of providing a daily dosage of estrogen and a daily dosage of progestin; a second vaginal ring capable of providing a total daily dosage of estrogen and progestin that is higher than that of the first vaginal ring and capable of providing a daily dosage of progestin that is less than twice that of the first vaginal ring; wherein the vaginal rings are capable of providing estrogen and progestin for a period of greater than 30 or 31 consecutive days. In some aspects of the invention, the second vaginal ring is capable of providing a daily dosage of estrogen that is equal to or higher than that of the first vaginal ring.

Some aspects of the invention provide a pharmaceutical kit comprising a first transdermal device capable of providing a daily dosage of estrogen and a daily dosage of progestin; a second transdermal device capable of providing a daily dosage of estrogen that is equal to or higher than that of the first transdermal device and capable of providing a total daily dosage of estrogen and progestin that is higher that of the first transdermal device; and a third transdermal device capable of providing a daily dosage of estrogen that is equal to or higher than that of the second transdermal device and capable of providing a total daily dosage of estrogen and progestin that is higher than that of the second transdermal device; wherein the transdermal devices are capable of providing estrogen and progestin for a period of greater than 30 or 31 consecutive days.

Other aspects of the invention provide a pharmaceutical kit comprising a first transdermal device capable of providing a daily dosage of estrogen and a daily dosage of progestin; a second transdermal device capable of providing a total daily dosage of estrogen and progestin that is higher than that of the first transdermal device and capable of providing a daily dosage of progestin that is less than twice that of the first transdermal device; wherein the transdermal devices are capable of providing estrogen and progestin for a period of greater than 30 or 31 consecutive days. In some aspects of the invention, the second transdermal device is capable of providing a daily dosage of estrogen that is equal to or higher than that of the first transdermal device.

Some aspects of the invention provide a pharmaceutical kit comprising a first oral dosage capable of providing a daily dosage of estrogen and a daily dosage of progestin; a second oral dosage capable of providing a daily dosage of estrogen that is equal to or higher than that of the first oral dosage and capable of providing a total daily dosage of estrogen and progestin that is higher than that of the first oral dosage; and a third oral dosage capable of providing a daily dosage of estrogen that is equal to or higher than that of the second oral dosage and capable of providing a total daily dosage of estrogen and progestin that is higher than that of the second oral dosage; wherein the oral dosages are capable of providing estrogen and progestin for a period of greater than 30 or 32 consecutive days.

Other aspects of the invention provide a pharmaceutical kit comprising a first oral dosage capable of providing a daily dosage of estrogen and a daily dosage of progestin; a second oral dosage capable of providing a total daily dosage of estrogen and progestin that is higher than that of the first oral dosage and capable of providing a daily dosage of progestin that is less than twice that of the first oral dosage; wherein the oral dosages are capable of providing estrogen and progestin for a period of greater than 30 or 31 consecutive days. In some aspects of the invention, the second oral dosage is capable of providing a daily dosage of estrogen that is equal to or higher than that of the first oral dosage. In some aspects of the invention, the pharmaceutical kit includes greater than 30 or 31 oral daily dosages.

The pharmaceutical kits of the invention can further include instructions for proper sequential administration in accordance with the regimens of the present invention.

The present invention is also directed to a method of delivering a pharmaceutical composition for an ascending-dose extended cycle regimen of the present invention to a patient in need thereof, the method comprising (a) registering in a computer readable medium the identity of a physician permitted to prescribe a pharmaceutical composition for an ascending-dose extended cycle regimen; (b) providing the patient with counseling information concerning the risks attendant to the pharmaceutical composition; (c) obtaining informed consent from the patient to receive the pharmaceutical composition despite the attendant risks; (d) registering the patient in a computer readable medium after obtaining their informed consent; and (e) permitting the patient access to the pharmaceutical composition.

The drug delivery methods of the present invention involve, inter alia, registering in a computer readable storage medium physicians who are qualified to prescribe the ascending-dose extended cycle regimen of the present invention. Once registered in the computer readable storage medium, the physician can be eligible to prescribe the pharmaceutical composition to a patient in need thereof. Generally speaking, in order to become registered in the computer readable storage medium, the physician may be required to comply with various aspects of, for example, providing patient education and counseling. The registration of the physician in the computer readable storage medium can be achieved by providing the physician, for example, by mail, facsimile transmission, or on-line transmission, with a registration card or form, preferably together with educational materials concerning the pharmaceutical composition of the present invention. The physician can complete the registration card or form by providing information requested therein, and the registration card or form can be returned to the manufacturer or distributor of the pharmaceutical composition of the present invention, or other authorized recipient of the registration materials, for example, by mail, facsimile transmission or on-line transmission. The physician's information in the registration card or form is then entered into the computer readable storage medium. Suitable computer readable storage media which can be employed for registration of the physicians (as well as patients, as discussed below) will be apparent to one of ordinary skill in the art, once in possession of the teaching of the present application.

In the course of examination of a patient, the physician may determine that the patient's condition can be improved by the administration of the pharmaceutical composition of the present invention. Prior to prescribing the pharmaceutical composition of the present invention, the physician can counsel the patient, for example, on the various risks and benefits associated with the pharmaceutical composition of the present invention. The patient can be provided full disclosure of all the known and suspected risks associated with the pharmaceutical composition of the present invention. Such counseling can be provided verbally, as well as in written form. In some embodiments, the physician can provide the patient with literature materials on the pharmaceutical composition of the present invention, such as product information, educational materials, and the like.

In addition to receiving counseling on the risks attendant to the pharmaceutical composition of the present invention, the methods of the invention further require the patient to fill out an informed consent form which is signed by the patient. Upon the completion of the informed consent form, the patient can be registered in a computer readable storage medium. The computer readable storage medium in which the patient is registered can be the same as, or different from, the computer readable storage medium in which the physician is registered.

The registration into one or more computer readable storage media of the physician and patient, according to the methods describe herein, provides a means to monitor and authorize access to the pharmaceutical composition of the present invention. Thus, the computer readable storage medium can serve to deny access to patients who fail to abide by the methods of the present invention. In some embodiments, access to the pharmaceutical composition of the present invention is in the form of a prescription, wherein the prescribing physician is registered in a computer readable storage medium, has provided counseling to the patient concerning the attendant risks of the pharmaceutical composition of the present invention, and has obtained informed consent from the patient, prior to prescribing the pharmaceutical composition of the present invention to the patient in need thereof.

All of the various aspects, embodiments and options described herein can be combined in any and all variations. The following examples are illustrative, but not limiting, of the method and compositions of the present invention. Other suitable modifications and adaptations of the variety of conditions and parameters normally encountered and obvious to those skilled in the art are within the spirit and scope of the invention. Thus, the breadth and scope of the present invention should not be limited by any of the above-described exemplary embodiments, but should be defined only in accordance with the following claims and their equivalents.

### Examples

### EXAMPLE 1

Tables 2-6 below show examples of ascending-dose extended cycle regimens for 2-month, 3-month, 4-month, 5-month, and 6-month cycles, respectively. All regimens below comprise three phases, wherein an equivalent of 20 µg of ethinyl estradiol is administered in the first phase, 25 µg of ethinyl estradiol is administered in the second phase, and 30 µg of ethinyl estradiol is administered in the third phase. For all regimens below, an equivalent of 150 µg of levonorgestrel is administered in all three phases. For each regimen below, two possibilities are provided for phase 3, one corresponding to an allowance of 7 days of placebo following the administration of the estrogen and progestin, and the other corresponding to an allowance of 3 days of placebo following the administration of the estrogen, and progestin.

**Table 2. Two-Month Ascending-Dose Extended Cycle Regimen**

| **Phases** | | **No. of days in each phase** | **Total estrogen dose (µg)** | **Total progestin dose (µg)** |
|---|---|---|---|---|
| 1 | | 14 | 280 | |
| 2 | | 21 | 525 | |
| 3 (with 7 days placebo | | 14 | 420 | |
| | or | | | |
| | 3 days placebo) | 18 | 540 | |
| | | | | |
| Total | | | | |
| (with 7 days placebo | | 49 (active) | 1225 | 7350 |
| | or | | | |
| | 3 days placebo) | 53 (active) | 1345 | 7950 |
| | | | | |
| Comparable Dose (2 cycles of 28-day monophasic 30 µg ethinyl estradiol oral contraceptive) | | 42 active | 1260 | 6300 |
| | | 14 placebo | | |

**Table 3. Three-Month Ascending-Dose Extended Cycle Regimen**

| **Phases** | | **No. of days in each phase** | **Total estrogen dose (µg)** | **Total progestin dose (µg)** |
|---|---|---|---|---|
| 1 | | 21 | 420 | |
| 2 | | 21 | 525 | |
| 3 (with 7 days placebo | | 42 | 1260 | |
| | or | | | |
| | 3 days placebo) | 46 | 1380 | |
| | | | | |
| Total | | | | |
| (with 7 days placebo | | 84 (active) | 2205 | 12600 |
| | or | | | |
| | 3 days placebo) | 88 (active) | 2325 | 13200 |
| | | | | |
| Comparable Dose | | 84 | 2520 | 12600 |
| (1 cycle of Seasonale®; 91 days) | | | | |

**Table 4. Four-Month Ascending-Dose Extended Cycle Regimen**

| **Phases** | | **No. of days in each phase** | **Total estrogen dose (µg)** | **Total progestin dose (µg)** |
|---|---|---|---|---|
| 1 | | 21 | 420 | |
| 2 | | 42 | 1050 | |
| 3 (with 7 days placebo | | 63 | 1890 | |
| | or | | | |
| | 3 days placebo) | 67 | 2010 | |
| | | | | |
| Total | | | | |
| (with 7 days placebo | | 105 (active) | 3360 | 15750 |
| | or | | | |
| | 3 days placebo) | 109 (active) | 3480 | 16350 |
| | | | | |
| Comparable Dose (4 cycles of 28-day monophasic 30 µg ethinyl estradiol oral contraceptive) | | 112 (84 active days) | 2520 | 12600 |

**Table 5. Five-Month Ascending-Dose Extended Cycle Regimen**

| **Phases** | | **No. of days in each phase** | **Total estrogen dose (µg)** | **Total progestin dose (µg)** |
|---|---|---|---|---|
| 1 | | 42 | 840 | |
| 2 | | 49 | 1225 | |
| 3 (with 7 days placebo | | 42 | 1260 | |
| | or | | | |
| | 3 days placebo) | 46 | 1380 | |
| | | | | |
| Total | | | | |
| (with 7 days placebo | | 133 (active) | 3325 | 19950 |
| | or | | | |
| | 3 days placebo) | 137 (active) | 3445 | 20550 |
| | | | | |
| Comparable Dose (5 cycles of 28-day monophasic 34 µg ethinyl estradiol oral contraceptive) | | 140 (105 active days) | 3150 | 15750 |

**Table 6. Six-Month Ascending-Dose Extended Cycle Regimen**

| **Phases** | | **No. of days in each phase** | **Total estrogen dose (µg)** | **Total progestin dose (µg)** |
|---|---|---|---|---|
| 1 | | 42 | 840 | |
| 2 | | 63 | 1575 | |
| 3 (with 7 days placebo | | | 2100 | |
| | or | 70 | | |
| | 3 days placebo) | 74 | 2220 | |
| | | | | |
| Total | | | | |
| (with 7 days placebo | | 175 (active) | 4515 | 26250 |
| | or | | | |
| | 3 days placebo) | 179 (active) | 4635 | 26850 |
| | | | | |
| Comparable Dose (2 cycle of Seasonale®; 91 days) | | 168 (active) | 5040 | 25200 |

**Table 7. One-Year Ascending-Dose Extended Cycle Regimen**

| **Phases** | | **No. of days In each phase** | **Total estrogen dose (µg)** | **Total progestin dose (µg)** |
|---|---|---|---|---|
| 1 | | 56 | 1124 | |
| 2 | | 175 | 4375 | |
| 3 (with 7 days placebo | | 126 | 3780 | |
| | or | | | |
| | 3 days placebo) | 130 | 3900 | |
| | | | | |
| Total | | | | |
| (with 7 days placebo | | 133 (active) | 9275 | 53550 |
| | or | | | |
| | 3 days placebo) | 137 (active) | 9395 | 54150 |

Application of the compounds, compositions and methods of the present invention for the medical or pharmaceutical uses described can be accomplished by any clinical, medical, and pharmaceutical methods and techniques as are presently or prospectively known to those skilled in the art. It will therefore be appreciated that the various embodiments which have been described above are intended to illustrate the invention and various changes and modifications can be made in the invention method without departing from the spirit or scope thereof

Having now fully described this invention, it will be understood to those of ordinary skill in the art that the same can be performed within a wide and equivalent range of conditions, compositions, and other parameters without affecting the scope of the invention or any embodiments thereof. All patents, patent applications, and publications cited herein are fully incorporated by reference herein in their entirety.

### NUMBERED EMBODIMENTS

### Numbered Embodiment 1

A method of contraception, the method comprising:
administering to a female in need thereof an estrogen and a progestin for a period of greater than 30 consecutive days,
wherein the estrogen and progestin are administered in at least three phases,
wherein a daily dosage of estrogen in a second phase is equal to or higher than a daily dosage of estrogen in a first phase,
wherein a daily dosage of estrogen in a third phase is equal to or higher than the daily dosage of estrogen in the second phase,
wherein a total daily dosage of estrogen and progestin in the second phase is higher than a total daily dosage of estrogen and progestin in the first phase, and
wherein a total daily dosage of estrogen and progestin in the third phase is higher than the total daily dosage of estrogen and progestin in the second phase.

### Numbered Embodiment 2

The method of numbered embodiment 1, wherein the daily dosages of progestin in the first, second, and third phases are equal to each other.

### Numbered Embodiment 3

The method of numbered embodiment 1, wherein a daily dosage of progestin in the second phase is higher than a daily dosage of progestin in the first phase, and a daily dosage of progestin in the third phase is higher than the daily dosage of progestin in the second phase.

### Numbered Embodiment 4

The method of numbered embodiment 1, wherein a daily dosage of progestin in the second phase is equal to a daily dosage of progestin in the first phase, and a daily dosage of progestin in the third phase is higher than the daily dosage of progestin in the second phase.

### Numbered Embodiment 5

The method of numbered embodiment 1, wherein a daily dosage of progestin in the second phase is higher than a daily dosage of progestin in the first phase, and a daily dosage of progestin in the third phase is equal to the daily dosage of progestin in the second phase.

### Numbered Embodiment 6

The method of numbered embodiment 1, wherein a daily dosage of progestin in the second phase is less than twice a daily dosage of progestin in the first phase.

### Numbered Embodiment 7

The method of numbered embodiment 1, wherein a daily dosage of progestin in the third phase is less than twice a daily dosage of progestin in the second phase.

### Numbered Embodiment 8

The method of numbered embodiment 2, wherein each of the daily dosages of progestin is the equivalent of 150 µg of levonorgestrel.

### Numbered Embodiment 9

The method of numbered embodiment 1, wherein the daily dosage of progestin in the first phase is the equivalent of 100 µg of levonorgestrel.

### Numbered Embodiment 10

The method of numbered embodiment 1, wherein the daily dosage of progestin in the second phase is the equivalent of 125 µg of levonorgestrel.

### Numbered Embodiment 11

The method of numbered embodiment 1, wherein the daily dosage of progestin in the third phase is the equivalent of 150 µg of levonorgestrel.

### Numbered Embodiment 12

The method of numbered embodiment 1, wherein the daily dosages of estrogen in the first, second, and third phases are equal to each other.

### Numbered Embodiment 13

The method of numbered embodiment 1, wherein the daily dosage of estrogen in the second phase is higher than the daily dosage of estrogen in the first phase, and the daily dosage of estrogen in the third phase is higher than the daily dosage of estrogen in the second phase.

### Numbered Embodiment 14

The method of numbered embodiment 1, wherein the daily dosage of estrogen in the second phase is equal to the daily dosage of estrogen in the first phase, and the daily dosage of estrogen in the third phase is higher than the daily dosage of estrogen in the second phase.

### Numbered Embodiment 15

The method of numbered embodiment 1, wherein the daily dosage of estrogen in the second phase is higher than the daily dosage of estrogen in the first phase, and the daily dosage of estrogen in the third phase is equal to the daily dosage of estrogen in the second phase.

### Numbered Embodiment 16

The method of numbered embodiment 1, wherein the daily dosage of estrogen in the second phase is less than twice the daily dosage of estrogen in the first phase.

### Numbered Embodiment 17

The method of numbered embodiment 1, wherein the daily dosage of estrogen in the third phase is less than twice the daily dosage of estrogen in the second phase.

### Numbered Embodiment 18

The method of numbered embodiment 1, wherein the daily dosage of estrogen in the first phase is the equivalent of 15 µg to 25 µg of ethinyl estradiol.

### Numbered Embodiment 19

The method of numbered embodiment 1, wherein the daily dosage of estrogen in the second phase is the equivalent of 20 µg to 30 µg of ethinyl estradiol.

### Numbered Embodiment 20

The method of numbered embodiment 1, wherein the daily dosage of estrogen in the third phase is the equivalent of 25 µg to 35 µg of ethinyl estradiol.

### Numbered Embodiment 21

The method of numbered embodiment 1, wherein the estrogen and progestin are administered orally and the daily dosage of estrogen is the equivalent of 15 µg to 50 µg of ethinyl estradiol and the daily dosage of progestin is the equivalent of 100 µg to 150 µg of levonorgestrel.

### Numbered Embodiment 22

The method of numbered embodiment 1, wherein the progestin is selected from the group consisting of levonorgestrel, norethindrone, norethindrone acetate, desogestrel, gestodene, dienogest, norgestimate, cyproterone acetate, norelgestromin, etonogestrel, progesterone, ethynodiol diacetate, norgestrel, trimegestone, medroxyprogesterone acetate, chlormadinone acetate, drospirenone, chlormadinone acetate, drospirenone, and esters, conjugates, and prodrugs thereof.

### Numbered Embodiment 23

The method of numbered embodiment 1, wherein the estrogen is selected from the group consisting of ethinyl estradiol, estradiol, estradiol acetate, estradiol valerate, mestranol, and esters, conjugates, and prodrugs thereof.

### Numbered Embodiment 24

The method of numbered embodiment 1, wherein the estrogen and progestin are administered for a period of 31 to 190 consecutive days.

### Numbered Embodiment 25

The method of numbered embodiment 1, wherein each phase is 7 to 84 days.

### Numbered Embodiment 26

The method of numbered embodiment 24, wherein the estrogen and progestin are administered for a period of 39 to 61 days.

### Numbered Embodiment 27

The method of numbered embodiment 26, wherein the estrogen and progestin are administered for a period of 42 to 60 days.

### Numbered Embodiment 28

The method of numbered embodiment 26, wherein the first phase is 7 to 21 days.

### Numbered Embodiment 29

The method of numbered embodiment 26, wherein the second phase is 14 to 28 days.

### Numbered Embodiment 30

The method of numbered embodiment 26, wherein the third phase is 7 to 25 days.

### Numbered Embodiment 31

The method of numbered embodiment 24, wherein the estrogen and progestin are administered for a period of 74 to 96 days.

### Numbered Embodiment 32

The method of numbered embodiment 31, wherein the estrogen and progestin are administered for a period of 77 to 95 days.

### Numbered Embodiment 33

The method of numbered embodiment 31, wherein the first phase is 14 to 28 days.

### Numbered Embodiment 34

The method of numbered embodiment 31, wherein the second phase is 14 to 28 days.

### Numbered Embodiment 35

The method of numbered embodiment 31, wherein the third phase is 35 to 53 days.

### Numbered Embodiment 36

The method of numbered embodiment 24, wherein the estrogen and progestin are administered for a period of 95 to 117 days.

### Numbered Embodiment 37

The method of numbered embodiment 36, wherein the estrogen and progestin are administered for a period of 98 to 116 days.

### Numbered Embodiment 38

The method of numbered embodiment 36, wherein the first phase is 14 to 28 days.

### Numbered Embodiment 39

The method of numbered embodiment 36, wherein the second phase is 35 to 49 days.

### Numbered Embodiment 40

The method of numbered embodiment 36, wherein the third phase is 56 to 74 days.

### Numbered Embodiment 41

The method of numbered embodiment 24, wherein the estrogen and progestin are administered for a period of 123 to 145 days.

### Numbered Embodiment 42

The method of numbered embodiment 41, wherein the estrogen and progestin are administered for a period of 126 to 144 days.

### Numbered Embodiment 43

The method of numbered embodiment 41, wherein the first phase is 35 to 49 days.

### Numbered Embodiment 44

The method of numbered embodiment 41, wherein the second phase is 42 to 56 days.

### Numbered Embodiment 45

The method of numbered embodiment 41, wherein the third phase is 35 to 53 days.

### Numbered Embodiment 46

The method of numbered embodiment 24, wherein the estrogen and progestin are administered for a period of 165 to 187 days.

### Numbered Embodiment 47

The method of numbered embodiment 46, wherein the estrogen and progestin are administered for a period of 168 to 186 days.

### Numbered Embodiment 48

The method of numbered embodiment 46, wherein the first phase is 35 to 49 days.

### Numbered Embodiment 49

The method of numbered embodiment 46, wherein the second phase is 56 to 70 days.

### Numbered Embodiment 50

The method of numbered embodiment 46, wherein the third phase is 63 to 81 days.

### Numbered Embodiment 51

The method of numbered embodiment 1, further comprising a hormone-free period.

### Numbered Embodiment 52

The method of numbered embodiment 51, wherein the hormone-free period is 2 to 10 consecutive days.

### Numbered Embodiment 53

The method of numbered embodiment 1, further comprising administering estrogen for a period of 2 to 10 consecutive days.

### Numbered Embodiment 54

The method of numbered embodiment 53, wherein the estrogen that is administered for the period of 2 to 10 consecutive days is in a daily dosage the equivalent of 5 µg to 50 µg of ethinyl estradiol.

### Numbered Embodiment 55

The method of numbered embodiment 1, further comprising administering a second active agent.

### Numbered Embodiment 56

The method of numbered embodiment 55, wherein the second active agent is administered (i) during a hormone-free period, (ii) in combination with an estrogen for a period of 2 to 10 consecutive days, (iii) continuously, (iv) intermittently, (v) one time, or (vi) once weekly.

### Numbered Embodiment 57

The method of numbered embodiment 55, wherein the second active agent is selected from the group consisting of calcium, iron, and folic acid.

### Numbered Embodiment 58

The method of numbered embodiment 1, further comprising administering an antidepressant.

### Numbered Embodiment 59

The method of numbered embodiment 58, wherein the antidepressant is administered (i) during a hormone-free period, (ii) in combination with an estrogen for a period of 2 to 10 consecutive days, (iii) continuously, (iv) intermittently, (v) one time, or (vi) once weekly.

### Numbered Embodiment 60

The method of numbered embodiment 58, wherein the antidepressant is a selective serotonin reuptake inhibitor (SSRI)

### Numbered Embodiment 61

The method of numbered embodiment 58, wherein the antidepressant is a selective serotonin and norepinephrine reuptake inhibitor (SSNRI).

### Numbered Embodiment 62

The method of numbered embodiment 1, further comprising monophasically administering an estrogen and a progestin.

### Numbered Embodiment 63

The method of numbered embodiment 62, wherein the estrogen and the progestin are monophasically administered for a period of greater than 30 consecutive days.

### Numbered Embodiment 64

The method of numbered embodiment 63, wherein the estrogen and the progestin are monophasically administered for a period of 60 to 110 consecutive days, followed by a hormone-free period of 2 to 10 consecutive days.

### Numbered Embodiment 65

The method of numbered embodiment 63, wherein the estrogen and the progestin are monophasically administered for a period of 60 to 110 consecutive days, followed by administering estrogen for a period of 2 to 10 consecutive days.

### Numbered Embodiment 66

The method of numbered embodiment 62, further comprising administering an antidepressant.

### Numbered Embodiment 67

The method of numbered embodiment 1, wherein the estrogen and progestin are in a dosage form selected from the group consisting of oral, transdermal, intravaginal, implantable pellet, and injectable liquid dosage forms.

### Numbered Embodiment 68

A method of contraception, the method comprising:
administering to a female in need thereof an estrogen and a progestin for a period of greater than 30 consecutive days,
wherein the estrogen and progestin are administered in at least two phases,
wherein a total daily dosage of estrogen and progestin in a second phase is higher than a total daily dosage of estrogen and progestin in a first phase, and
wherein a daily dosage of progestin in the second phase is less than twice a daily dosage of progestin in the first phase.

### Numbered Embodiment 69

The method of numbered embodiment 68, wherein the daily dosages of progestin in the first and second phases are equal to each other.

### Numbered Embodiment 70

The method of numbered embodiment 68, wherein the daily dosage of progestin in the second phase is higher than the daily dosage of progestin in the first phase.

### Numbered Embodiment 71

The method of numbered embodiment 69, wherein each of the daily dosages of progestin is the equivalent of 150 µg of levonorgestrel.

### Numbered Embodiment 72

The method of numbered embodiment 68, wherein the daily dosage of progestin in the first phase is the equivalent of 100 µg of levonorgestrel.

### Numbered Embodiment 73

The method of numbered embodiment 68, wherein the daily dosage of progestin in the second phase is the equivalent of 125 µg of levonorgestrel.

### Numbered Embodiment 74

The method of numbered embodiment 68, wherein the daily dosages of estrogen in the first and second phases are equal to each other.

### Numbered Embodiment 75

The method of numbered embodiment 68, wherein a daily dosage of estrogen in the second phase is higher than a daily dosage of estrogen in the first phase.

### Numbered Embodiment 76

The method of numbered embodiment 68, wherein a daily dosage of estrogen in the second phase is less than twice a daily dosage of estrogen in the first phase.

### Numbered Embodiment 77

The method of numbered embodiment 68, wherein a daily dosage of estrogen in the first phase is the equivalent of 15 µg to 25 µg of ethinyl estradiol.

### Numbered Embodiment 78

The method of numbered embodiment 68, wherein a daily dosage of estrogen in the second phase is the equivalent of 20 µg to 30 µg of ethinyl estradiol.

### Numbered Embodiment 79

The method of numbered embodiment 68, wherein the estrogen and progestin are administered orally and the daily dosage of estrogen is the equivalent of 15 µg to 50 µg of ethinyl estradiol and the daily dosage of progestin is the equivalent of 100 µg to 150 µg of levonorgestrel.

### Numbered Embodiment 80

The method of numbered embodiment 68, wherein the progestin is selected from the group consisting of levonorgestrel, norethindrone, norethindrone acetate, desogestrel, gestodene, dienogest, norgestimate, cyproterone acetate, norelgestromin, etonogestrel, progesterone, ethynodiol diacetate, norgestrel, trimegestone, medroxyprogesterone acetate, chlormadinone acetate, drospirenone, and esters, conjugates, and prodrugs thereof.

### Numbered Embodiment 81

The method of numbered embodiment 68, wherein the estrogen is selected from the group consisting of ethinyl estradiol, estradiol, estradiol acetate, estradiol valerate, mestranol, and esters, conjugates, and prodrugs thereof.

### Numbered Embodiment 82

The method of numbered embodiment 68, wherein the estrogen and progestin are administered for a period of 31 to 190 consecutive days.

### Numbered Embodiment 83

The method of numbered embodiment 68, wherein each phase is 7 to 84 days.

### Numbered Embodiment 84

The method of numbered embodiment 82, wherein the estrogen and progestin are administered for a period of 39 to 61 days.

### Numbered Embodiment 85

The method of numbered embodiment 84, wherein the estrogen and progestin are administered for a period of 42 to 60 days.

### Numbered Embodiment 86

The method of numbered embodiment 82, wherein the estrogen and progestin are administered for a period of 74 to 96 days.

### Numbered Embodiment 87

The method of numbered embodiment 86, wherein the estrogen and progestin are administered for a period of 77 to 95 days.

### Numbered Embodiment 88

The method of numbered embodiment 82, wherein the estrogen and progestin are administered for a period of 95 to 117 days.

### Numbered Embodiment 89

The method of numbered embodiment 88, wherein the estrogen and progestin are administered for a period of 98 to 116 days.

### Numbered Embodiment 90

The method of numbered embodiment 82, wherein the estrogen and progestin are administered for a period of 123 to 145 days.

### Numbered Embodiment 91

The method of numbered embodiment 90, wherein the estrogen and progestin are administered for a period of 126 to 144 days.

### Numbered Embodiment 92

The method of numbered embodiment 82, wherein the estrogen and progestin are administered for a period of 165 to 187 days.

### Numbered Embodiment 93

The method of numbered embodiment 92, wherein the estrogen and progestin are administered for a period of 168 to 186 days.

### Numbered Embodiment 94

The method of numbered embodiment 68, further comprising a hormone-free period.

### Numbered Embodiment 95

The method of numbered embodiment 94, wherein the hormone-free period is 2 to 10 consecutive days.

### Numbered Embodiment 96

The method of numbered embodiment 68, further comprising administering estrogen for a period of 2 to 10 consecutive days.

### Numbered Embodiment 97

The method of numbered embodiment 96, wherein the estrogen that is administered for the period of 2 to 10 consecutive days is in a daily dosage that is the equivalent of 5 µg to 50 µg of ethinyl estradiol.

### Numbered Embodiment 98

The method of numbered embodiment 68, further comprising administering a second active agent.

### Numbered Embodiment 99

The method of numbered embodiment 98, wherein the second active agent is administered (i) during a hormone-free period, (ii) in combination with an estrogen for a period of 2 to 10 consecutive days, (iii) continuously, (iv) intermittently, (v) one time, or (vi) once weekly.

### Numbered Embodiment 100

The method of numbered embodiment 98, wherein the second active agent is selected from the group consisting of calcium, iron, and folic acid.

### Numbered Embodiment 101

The method of numbered embodiment 68, further comprising administering an antidepressant.

### Numbered Embodiment 102

The method of numbered embodiment 101, wherein the antidepressant is administered (i) during a hormone-free period, (ii) in combination with an estrogen for a period of 2 to 10 consecutive days, (iii) continuously, (iv) intermittently, (v) one time, or (vi) once weekly.

### Numbered Embodiment 103

The method of numbered embodiment 101, wherein the antidepressant is a selective serotonin reuptake inhibitor (SSRI).

### Numbered Embodiment 104

The method of numbered embodiment 101, wherein the antidepressant is a selective serotonin and norepinephrine reuptake inhibitor (SSNRI).

### Numbered Embodiment 105

The method of numbered embodiment 68, further comprising monophasically administering an estrogen and a progestin.

### Numbered Embodiment 106

The method of numbered embodiment 105, wherein the estrogen and the progestin are monophasically administered for a period of greater than 30 consecutive days,

### Numbered Embodiment 107

The method of numbered embodiment 106, wherein the estrogen and the progestin are monophasically administered for a period of 60 to 110 consecutive days, followed by a hormone-free period of 2 to 10 consecutive days.

### Numbered Embodiment 108

The method of numbered embodiment 106, wherein the estrogen and the progestin are monophasically administered for a period of 60 to 110 consecutive days, followed by administering estrogen for a period of 2 to 10 consecutive days.

### Numbered Embodiment 109

The method of numbered embodiment 105, further comprising administering an antidepressant.

### Numbered Embodiment 110

The method of numbered embodiment 68, wherein the estrogen and progestin are in a dosage form selected from the group consisting of oral, transdermal, intravaginal, implantable pellet, and injectable liquid dosage forms.

### Numbered Embodiment 111

A pharmaceutical kit comprising:
a first vaginal ring capable of providing a daily dosage of estrogen and a daily dosage of progestin;
a second vaginal ring capable of providing a daily dosage of estrogen that is equal to or higher than that of the first vaginal ring and capable of providing a total daily dosage of estrogen and progestin that is higher than that of the first vaginal ring; and
a third vaginal ring capable of providing a daily dosage of estrogen that is equal to or higher than that of the second vaginal ring and capable of providing a total daily dosage of estrogen and progestin that is higher than that of the second vaginal ring;
wherein the vaginal rings are capable of providing estrogen and progestin for a period of greater than 30 consecutive days.

### Numbered Embodiment 112

A pharmaceutical kit comprising:
a first vaginal ring capable of providing a daily dosage of estrogen and a daily dosage of progestin;
a second vaginal ring capable of providing a total daily dosage of estrogen and progestin that is higher than that of the first vaginal ring and capable of providing a daily dosage of progestin that is less than twice that of the first vaginal ring;
wherein the vaginal rings are capable of providing estrogen and progestin for a period of greater than 30 consecutive days.

### Numbered Embodiment 113

The pharmaceutical kit of numbered embodiment 112, wherein the second vaginal ring is capable of providing a daily dosage of estrogen that is equal to or higher than that of the first vaginal ring.

### Numbered Embodiment 114

A pharmaceutical kit comprising:
a first transdermal device capable of providing a daily dosage of estrogen and a daily dosage of progestin;
a second transdermal device capable of providing a daily dosage of estrogen that is equal to or higher than that of the first transdermal device and capable of providing a total daily dosage of estrogen and progestin that is higher than that of the first transdermal device; and
a third transdermal device capable of providing a daily dosage of estrogen that is equal to or higher than that of the second transdermal device and capable of providing a total daily dosage of estrogen and progestin that is higher than that of the second transdermal device;
wherein the transdermal devices are capable of providing estrogen and progestin for a period of greater than 30 consecutive days.

### Numbered Embodiment 115

A pharmaceutical kit comprising:
a first transdermal device capable of providing a daily dosage of estrogen and a daily dosage of progestin;
a second transdermal device capable of providing a total daily dosage of estrogen and progestin that is higher than that of the first transdermal device and capable of providing a daily dosage of progestin that is less than twice that of the first transdermal device;
wherein the transdermal devices are capable of providing estrogen and progestin for a period of greater than 30 consecutive days.

### Numbered Embodiment 116

The pharmaceutical kit of numbered embodiment 115, wherein the second transdermal device is capable of providing a daily dosage of estrogen that is equal to or higher than that of the first transdermal device.

### Numbered Embodiment 117

A pharmaceutical kit comprising:
a first oral dosage capable of providing a daily dosage of estrogen and a daily dosage of progestin;
a second oral dosage capable of providing a daily dosage of estrogen that is equal to or higher than that of the first oral dosage and capable of providing a total daily dosage of estrogen and progestin that is higher than that of the first oral dosage; and
a third oral dosage capable of providing a daily dosage of estrogen that is equal to or higher than that of the second oral dosage and capable of providing a total daily dosage of estrogen and progestin that is higher than that of the second oral dosage;
wherein the oral dosages are capable of providing estrogen and progestin for a period of greater than 30 consecutive days.

### Numbered Embodiment 118

A pharmaceutical kit comprising:
a first oral dosage capable of providing a daily dosage of estrogen and a daily dosage of progestin,
a second oral dosage capable of providing a total daily dosage of estrogen and progestin that is higher than that of the first oral dosage and capable of providing a daily dosage of progestin that is less than twice that of the first oral dosage;
wherein the oral dosages are capable of providing estrogen and progestin for a period of greater than 30 consecutive days.

### Numbered Embodiment 119

The pharmaceutical kit of numbered embodiment 118, wherein the second oral dosage is capable of providing a daily dosage of estrogen that is equal to or higher than that of the first oral dosage.

## Claims

1. A method of contraception, the method comprising:
administering to a female in need thereof a dosage comprising estrogen and progestin for a period of 74 to 96 consecutive days,
wherein the dosage comprising estrogen and progestin is administered in at least two phases,
wherein the total daily dosage of estrogen and progestin in the second phase is higher than the total daily dosage of estrogen and progestin in the first phase, and
wherein the daily dosage of progestin in the second phase is less than twice the daily dosage of progestin in the first phase.

2. The method of claim 1, wherein the daily dosage of estrogen in the second phase is equal to or higher than the daily dosage of estrogen in the first phase.

3. The method of claim 1, wherein the daily dosage of estrogen in the second phase is less than twice the daily dosage of estrogen in the first phase.

4. The method of claim 1, wherein the daily dosage of estrogen in the first phase is equivalent to 15 µg to 25 µg of ethinyl estradiol.

5. The method of claim 1, wherein the daily dosage of estrogen in the second phase is equivalent to 20 µg to 30 µg of ethinyl estradiol.

6. The method of claim 1, wherein the daily dosages of progestin in the first and second phases are equal to each other.

7. The method of claim 1, wherein the daily dosage of progestin in the second phase is higher than the daily dosage of progestin in the first phase.

8. The method of claim 1, wherein the daily dosage of progestin in each of the two phases is equivalent to 150 µg of levonorgestrel.

9. The method of claim 1, wherein the estrogen and progestin are administered orally and the daily dosage of estrogen is the equivalent of 15 µg to 50 µg of ethinyl estradiol and the daily dosage of progestin is the equivalent of 100 µg to 150 µg of levonorgestrel.

10. The method of claim 1, wherein the progestin is selected from the group consisting of levonorgestrel, norethindrone, norethindrone acetate, desogestrel, gestodene, dienogest, norgestimate, cyproterone acetate, norelgestromin, etonogestrel, progesterone, ethynodiol diacetate, norgestrel, trimegestone, medroxyprogesterone acetate, chlormadinone acetate, drospirenone, and esters, conjugates, and prodrugs thereof.

11. The method of claim 1, wherein the estrogen is selected from the group consisting of ethinyl estradiol, estradiol, estradiol acetate, estradiol valerate, mestranol, and esters, conjugates, and prodrugs thereof.

12. The method of claim 1, wherein the dosage comprising estrogen and progestin is administered in at least three phases, wherein the daily dosage of estrogen in the second phase is equal to or higher than the daily dosage of estrogen in the first phase, wherein the daily dosage of estrogen in the third phase is equal to or higher than the daily dosage of estrogen in the second phase, and wherein the total daily dosage of estrogen and progestin in the third phase is higher than the total daily dosage of estrogen and progestin in the second phase.

13. The method of claim 12, wherein the first phase is 14 to 28 days, the second phase is 14 to 28 days, and the third phase is 35 to 53 days.

14. The method of claim 13, wherein the first phase is 21 days, the second phase is 21 days, and the third phase is 42 days.

15. The method of claim 14, wherein the daily dosage of estrogen in the first phase is 20 µg of ethinyl estradiol, the daily dosage of estrogen in the second phase is 25 µg of ethinyl estradiol, and the daily dosage of estrogen in the third phase is 30 µg of ethinyl estradiol, and wherein the daily dosage of progestin in each of the three phases is 150 µg of levonorgestrel.

16. The method of claim 1, further comprising a hormone-free period.

17. The method of claim 16, wherein the hormone-free period is 2 to 10 consecutive days.

18. The method of claim 1, further comprising administering to the female a daily dosage consisting essentially of estrogen for a period of 2 to 10 consecutive days following the administration of the dosage comprising estrogen and progestin for 74 to 96 consecutive days.

19. The method of claim 18, wherein the dosage of estrogen that is administered for the period of 2 to 10 consecutive days is equivalent to 5 µg to 30 µg of ethinyl estradiol.

20. The method of claim 19, wherein the dosage of estrogen that is administered for the period of 2 to 10 consecutive days is equivalent to 10 µg of ethinyl estradiol.

21. The method of claim 15, further comprising administering to the female a daily dosage consisting essentially of estrogen for 7 consecutive days.

22. The method of claim 1, further comprising administering a second active agent.

23. The method of claim 22, wherein the second active agent is administered (i) during a hormone-free period, (ii) in combination with a dosage consisting essentially of an estrogen for a period of 2 to 10 consecutive days following the administration of the dosage comprising estrogen and progestin for 74 to 96 consecutive days, (iii) continuously, (iv) intermittently, (v) one time, or (vi) once weekly.

24. The method of claim 22, wherein the second active agent is selected from the group consisting of calcium, iron, and folic acid.

25. A pharmaceutical kit comprising 74 to 96 daily dosages in sequential order for administration in at least two phases, each daily dosage comprising estrogen and progestin,
wherein the total daily dosage of estrogen and progestin in the second phase is higher than the total daily dosage of estrogen and progestin in the first phase, and
wherein the daily dosage of progestin in the second phase is less than twice the daily dosage of progestin in the first phase.

26. The pharmaceutical kit of claim 25, wherein the daily dosage of estrogen in the second phase is equal to or higher than the daily dosage of estrogen in the first phase

27. The pharmaceutical kit of claim 25, wherein the daily dosages of progestin in the first and second phases are equal to each other.

28. The pharmaceutical kit of claim 25, wherein the daily dosage of progestin in the second phase is higher than the daily dosage of progestin in the first phase.

29. The pharmaceutical kit of claim 25, wherein the daily dosage of progestin is equivalent to 100 µg to 150 µg of levonorgestrel.

30. The pharmaceutical kit of claim 25, wherein the daily dosage of progestin in each of the two phases is equivalent to 150 µg of levonorgestrel.

31. The pharmaceutical kit of claim 25, wherein the progestin is selected from the group consisting of levonorgestrel, norethindrone, norethindrone acetate, desogestrel, gestodene, dienogest, norgestimate, cyproterone acetate, norelgestromin, etonogestrel, progesterone, ethynodiol diacetate, norgestrel, trimegestone, medroxyprogesterone acetate, chlormadinone acetate, drospirenone, and esters, conjugates, and prodrugs thereof.

32. The pharmaceutical kit of claim 25, wherein the estrogen is selected from the group consisting of ethinyl estradiol, estradiol, estradiol acetate, estradiol valerate, mestranol, and esters, conjugates, and prodrugs thereof.

33. The pharmaceutical kit of claim 25, wherein the kit contains dosages comprising estrogen and progestin for administration in at least three phases, wherein the daily dosage of estrogen in the second phase is equal to or higher than the daily dosage of estrogen in the first phase, wherein the daily dosage of estrogen in the third phase is equal to or higher than the daily dosage of estrogen in the second phase, and wherein the total daily dosage of estrogen and progestin in the third phase is higher than the total daily dosage of estrogen and progestin in the second phase.

34. The pharmaceutical kit of claim 33, wherein the kit contains 14 to 28 daily dosages in the first phase, 14 to 28 daily dosages in the second phase, and 35 to 53 daily dosages in the third phase.

35. The pharmaceutical kit of claim 34, wherein the kit contains 21 daily dosages in the first phase, 21 daily dosages in the second phase, and 42 daily dosages in the third phase.

36. The pharmaceutical kit of claim 35, wherein the daily dosage of estrogen in the first phase is 20 µg of ethinyl estradiol, the daily dosage of estrogen in the second phase is 25 µg of ethinyl estradiol, and the daily dosage of estrogen in the third phase is 30 µg of ethinyl estradiol, and wherein the daily dosage of progestin in each of the three phases is 150 µg of levonorgestrel.

37. The pharmaceutical kit of claim 25, further comprising 2 to 10 daily dosages consisting essentially of estrogen for administration following the administration of 74 to 96 dosages comprising estrogen and progestin.

38. The pharmaceutical kit of claim 37, wherein each of the 2 to 10 daily dosages consisting essentially of estrogen is equivalent to 5 µg to 30 µg of ethinyl estradiol.

39. The pharmaceutical kit of claim 25, wherein the daily dosage of estrogen in the first phase is 20 µg of ethinyl estradiol, the daily dosage of estrogen in the second phase is 25 µg of ethinyl estradiol, and wherein the daily dosage of progestin in each of the two phases is 150 µg of levonorgestrel.

40. The pharmaceutical kit of claim 25, further comprising 7 daily dosages consisting essentially of 10 µg of ethinyl estradiol.
